# EUROPEAN PATENT APPLICATION

(11) **EP 1 745 776 A1**
(43) Date of publication of application: **24.01.2007**
(21) Application number: 06117471.0
(22) Date of filing: 19.07.2006
(51) Int. Cl.: A61K 31/132, A61P 25/28

(54) **Aminoalcohols as therapeutic compounds**

(30) Priority: 20.07.2005 CH 12102005
(71) Applicant: Speedel Experimenta AG, 4123 Allschwil (CH)
(72) Inventor: Herold, Peter, 4055, Basel (CH); Stutz, Stefan, 4053, Basel (CH); Tschinke, Vincenzo, 4102, Binningen (CH); Stojanovic, Aleksandar, 4055, Basel (CH); Marti, Christiane, 5400, Baden (CH); Quirmbach, Michael, 4054, Basel (CH); Schumacher, Christoph, 4126, Bettingen (CH)
(74) Representative: Dannappel, Hans-Jochen

(57) **Abstract**

Use of compounds of the general formula (I) 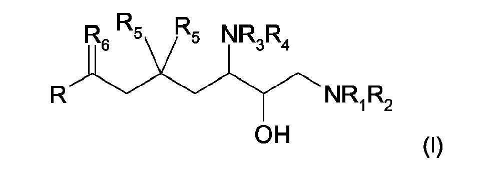 in which R, R₁, R₂, R₃, R₄, R₅ and R₆ have the definitions illustrated in detail in the description, as beta-secretase, cathepsin D, plasmepsin II and/or HIV protease inhibitors.

## Description

The present invention relates to the use of aminoalcohols as beta-secretase-, cathepsin D-, plasmepsin II- and/or HIV-protease-inhibitors.

With regard to beta-secretase-, cathepsin D-, plasmepsin II- and/or HIV-protease-inhibition, there is still a need for highly potent active ingredients. In this context, the improvement of the pharmacokinetic properties is at the forefront. These properties directed towards better bioavailability are, for example, absorption, metabolic stability, solubility or lipophilicity.

### Alzheimer Disease aspartyl protease: Beta-Secretase

Alzheimer's disease (AD) is a progressive degenerative disease of the brain. The symptoms of AD include progressive memory loss, language difficulty and ultimately loss of basic neural function and death. The biomarkers in the central nervous system for AD include amyloid plaques, intracellular neurofibrillary tangles and activated microglia. The appearance of these three markers is likely to contribute to the neuronal cell death and memory loss observed in AD.

Beta-amyloid is a defining feature of AD and now believed to be a causative precursor in the development of the disease. Amyloidogenic plaques and vascular amyloid angiopathy also characterize the brains of individuals with Trisomy 21 (Down's Syndrome), Hereditary Cerebral Hemorrhage with Amloidosis of the Dutch-Type (HCHWA-D) and other neurodegenerative disorders.

Beta-amyloid plaques are predominantly composed of amyloid beta peptide (A-beta, also sometimes designated betaA4). The A-beta peptide is derived by proteolysis of the beta amyloid precursor protein (APP). Beta-APP is processed by three distinct ordered enzymatic activities. The bulk of beta-APP is processed via alpha-secretase in a non-amyloidogenic pathway. A small fraction of beta-APP is cleaved by beta-secretase activity to generate the membrane-bound C-terminal fragment C99. Gamma-secretase cleaves C99 to generate the amyloidogenic A-beta peptide of 39-42 amino acids. The aspartyl protease activity of beta-secretase has been disclosed using varied nomenclature, including BACE (beta-site APP cleaving enzyme), Asp and memapsin.

The significance of beta-secretase cleavage of beta-APP as a critical step in the generation of AD is underscored by the observation that human mutations at the beta-secretase cleavage subsites (Swedish mutations) of beta-APP lead to increased A-beta production and early onset familial AD. Furthermore, BACE1- knockout mice fail to produce A-beta peptide and present a normal phenotype. When crossed with transgenic mice that overexpress APP, the progeny show reduced amounts of A-beta in brain extracts as compared with control animals. This evidence supports the proposal that inhibition of beta-secretase activity and reduction of A-beta peptide deposits in the brain provides a therapeutic strategy for the treatment of AD and other beta amyloid disorders as described by Verdile et al. (2004) in Pharmacol. Res 50, 397-409.

Compounds that are effective inhibitors of beta-secretase may inhibit beta-secretase-mediated cleavage of APP and the production of A-beta peptide. The pharmacological inhibition of A-beta peptide generation may reduce amyloid beta deposits, respectively the formation of plaques. Beta-secretase inhibiting compounds as discussed by Thompson et al. (2005) in Curr. Pharm. Des. 11, 3383-3404 are therefore useful to treat or to prevent diseases that are characterized by amyloid beta deposits or plaques such as AD.

The present invention also relates to methods of treating subjects who have, or in preventing subjects from developing a disease or condition selected from the group consisting of AD, for helping prevent or delay the onset of AD, for helping to slow the proression of AD, for treating subjects with mild cognitive impairment (MCI) and preventing or delaying the onset of AD in those who could progress form MCI to AD, for treating Down's syndrome, for treating humans who have HCHWAD, for treating cerebral amyloid angiopathy, and for treating degenerative dementias.

### Alzheimner's Disease aspartyl protease: Cathepsin D

Human cathepsin D is an intracellular aspartic peptidase found mainly in lysosomes. It has a number of housekeeping functions, including the degradation of cellular and phagocytosed proteins. The enzymes may be involved in a variety of disease states, including cancer and Alzheimer's disease (AD). Clinical studies have shown that cathepsin D is overexpressed in breast cancer cells and this seems to be associated with an increased risk for metastasis due to enhanced cell growth. Cathepisn D is also thought to be involved in formation of the beta-amyloid peptide in AD. Recently, several genetic association studies linked cathepsin D with amyloid pathology and Alzheimer's disease as described for example by Davidson et al., (2006) in J. Neurol. Neurosurg. Psychiatry 77, 515-517. The availability of selective and potent inhibitors will help to further define the role of cathepsin D in disease and possibly lead to therapeutic agents.

### Malaria Aspartyl Protease: Plasmepsin I and II

Malaria is considered as one of the most serious infectious diseases in the world, affecting approximately 500 million people. The disease is spread by the anopheles mosquito that is mostly found in tropical regions. The species plasmodium falciparum is responsible for more than 95% of malaria-related morbidity and mortality. Increasingly, plasmodium falciparum is becoming resistant to existing therapies such as chloroquine, mefloquine and sulfadoxime/pyrimethamine. Thus there is an urgent need for new treatments.

In the erythrocytic stage of the parasite's life cycle the parasite invades the red blood cells of its host consuming up to 80% of the hemoglobin as a source of nutrients for growth and development. Hemoglobin degradation takes place in an acidic vacuole of the parasite and many of the current antimalarial drugs appear to disrupt important vacuolar functions. The food vacuole contains aspartic, cysteine and metallo-proteases, which are all considered to play a role in the process of hemoglobin degradation. At least 10 genes encoding aspartic proteases have been identified in the plasmodium genome. Four of the aspartic proteases have been localized in the acidic food vacuole of the parasite, namely plasmepsin I, II, IV and HAP, a histo-aspartic protease. Inhibitors of plasmepsin I and II have shown efficacy in cell and animal models of malaria, indicating that these enzymes may represent targets for drug discovery as described for example by Coombs et al. (2001) Trends Parasitol 17, 532-537. Indeed, a non-selective inhibitor of aspartic proteases, pepstatin, inhibits the growth of plasmodium falciparum in vitro. Similar results have been obtained with analogs of pepstatin or with immunodeficiency virus protease inhibitors indicating that inhibition of aspartic proteases interferes with the life cycle of plasmodium falciparum as noted for example by Andrews et al. (2006) in Antimicrob. Agents Chemother 50, 639-648.

The present invention relates to the identification of low molecular weight, non-peptidic inhibitors of the plasmodium falciparum protease plasmepsin II or other related aspartic proteases to treat and/or to prevent malaria.

### HIV aspartyl protease: HIV-1 peptidase

First reported in 1981 in a small number of patients, Acquired immunodeficiency syndrome (AIDS) has now become a major epidemic with more than 38 million people infected worldwide, including approximately 1 million in the United States, 580,000 in Western Europe and more than 25 million in Sub-Saharan Africa (http://www.unaids.org). Since AIDS was first clinically identified, scientific and therapeutic progress has been extraordinary. However, AIDS remains out of control, especially in developing countries.

The prognosis of AIDS patients who have full access to current therapies has completely changed since the first cases of AIDS were reported. Today, the median survival for HIV-positive patients receiving treatment exceeds 8 years. The life expectancy for AIDS patients was less than 1 year before AZT was introduced in 1987. This dramatic change is due to the development of effective therapies, to early detection of HIV-positive individuals, and to a sustained effort to analyze and understand viral-resistance mechanisms, which can be overcome by rational drug development and combination therapy.

FDA-approved therapies target three steps of the HIV life cycle: reverse transcription, proteolytic maturation and fusion. Triple therapy, commonly referred to as HIGHLY ACTIVE ANTIRETROVIRAL THERAPY (HAART), is now the standard for treatment. It consists of a protease inhibitor or a non-nucleoside reverse transcriptase inhibitor in combination with two nucleoside reverse transcriptase inhibitors.

Translation of human immunodeficiency virus type-1 (HIV-1) genomic RNA results in the production of two polyprotein precursors, Gag and Gag-Pol. The 55-kDa Gag precursor contains the structural proteins and the 160-kDa Gag-Pol polyprotein contains the functional viral enzymes protease, reverse transcriptase, and integrase. Gag and Gag-Pol polyproteins are transported to the plasma membrane where assembly of type-C retroviruses and lentiviruses typically occurs. During particle assembly, the viral protease cleaves the Gag and Gag-Pol precursors into the structural and functional proteins required for viral replication. The protease activity within the cytoplasma of infected cells allows for the formation of virions which can be released from the cell in the last stages of budding.

The mature HIV-1 protease is an obligatory dimer of identical 11-kDa subunits, each contributing one of the two catalytic aspartic residues. In contrast, the cell-derived members of the aspartic protease family are monomeric enzymes with two Asp-Thr-Gly-containing domains. The unique dimeric structure of the retroviral protease is mainly stabilized by an antiparallel beta-sheet formed by the interdigitation of the amino- and carboxyl-terminal betastrands of each monomer.

The activation of HIV-1 protease i.e. the dimerization and autocatalytic release from Gag-Pol, is a critical step in the viral life cycle. Inhibition of protease activation causes a severe defect in Gag polyprotein processing and a complete loss of viral infectivity.

As such, the viral protease has become a target for HIV therapeutics, resulting in many HIV protease inhibitors reaching clinical trials as reviewed by Rana et al. (1999) in Pharmacotherapy 19, 35-59 and Morse et al., (2006) in Lancet Infect. Dis. 6, 215-225. Most of these drugs are substrate-based inhibitors, whose design has been facilitated by an abundance of crystal structure data for both the native enzyme and enzyme-inhibitor complexes. Additionally, there are now extensive biochemical data detailing both the catalytic mechanism and the molecular basis for substrate selection.

Firstly, the present invention relates to the use as beta-secretase-, cathepsin D-, plasmepsin II- and/or HIV-protease-inhibitors of compounds of the general formula 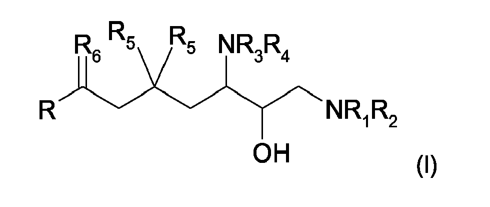 where
R₁ is a) hydrogen, amino or hydroxyl; or
is b) C₁-C₈-alkyl, C₃-C₈-cycloalkyl, C₁-C₈-alkanoyl, C₁-C₈-alkoxycarbonyl, aryl-C₀-C₄-alkyl or heterocyclyl-C₀-C₄-alkyl, which radicals may be substituted by 1-4, C₁-C₈-alkyl, halogen, cyano, oxide, oxo, trifluoromethyl, C₁-C₈-alkoxy, C₁-C₈-alkoxycarbonyl, aryl or heterocyclyl;
R₂ is a) C₁-C₈-alkyl, C₃-C₈-cycloalkyl, C₁-C₈-alkylsulphonyl, C₃-C₈-cycloalkylsulphonyl, aryl-C₀-C₈-alkylsulphonyl, heterocyclylsulphonyl, C₃-C₈-cycloalkyl-C₁-C₈-alkanoyl, aryl-C₁-C₈₋alkanoyl, aryl-C₃-C₈-cycloalkanoyl, C₁-C₈-alkanoyl, C₁-C₈-alkoxycarbonyl, optionally N-mono- or N,N-di-C₁-C₈-alkylated carbamoyl-C₀-C₈-alkyl, aryl-C₀-C₄-alkyl or heterocyclyl-C₀-C₄-alkyl, which radicals may be substituted by 1-4 C₁-C₈-alkyl, C₃-C₁₂-cycloalkyl, C₃-C₈-cycloalkoxy, amino, C₁-C₆-alkylamino, di-C₁-C₆-alkylamino, C₁-C₆-alkanoylamino, C₁-C₆-alkoxycarbonylamino, halogen, oxo, cyano, hydroxyl, oxide, trifluoromethyl, C₁-C₈-alkoxy, optionally N-mono- or N,N-di-C₁-C₈-alkylated carbamoyl-C₀-C₈-alkyl, optionally esterified carboxyl, C₁₋C₆-alkylenedioxy, aryl or heterocyclyl; or
is b) together with R₁ and the nitrogen atom to which they are bonded a saturated or partly unsaturated 4-8-membered heterocyclic ring which may contain an additional nitrogen, oxygen or sulphur atom or a -SO- or -SO2- group, in which case the additional nitrogen atom may optionally be substituted by C₁-C₈-alkyl, C₁-C₈-alkanoyl, C₁-C₈-alkoxycarbonyl, aryl or heterocyclyl radicals, and this heterocyclic ring may be part of a bicyclic or tricyclic ring system having a total of up to 16 members and the second ring may also contain a nitrogen, oxygen or sulphur atom or a -SO- or -SO2- group, and the nitrogen atom in the second ring may optionally be substituted by C₁-C₈-alkyl, C₁-C₈-alkanoyl, C₁-C₈-alkoxycarbonyl, aryl or heterocyclyl radicals and all ring systems mentioned may be substituted by 1-4 C₁-C₈-alkyl, C₃-C₈-cycloalkyl, C₁-C₈-alkylsulphonyl, C₃-C₈-cycloalkylsulphonyl, aryl-C₀-C₈-alkylsulphonyl, heterocyclylsulphonyl, C₃-C₈-cycloalkyl-C₁-C₈-alkanoyl, aryl-C₁-C₈-alkanoyl, C₁-C₈-alkanoyl, C₁₋C₈-alkoxycarbonyl, optionally N-mono- or N,N-di-C₁-C₈-alkylated carbamoyl-C₀-C₈-alkyl, halogen, hydroxyl, oxide, oxo, trifluoromethyl, C₁-C₈-alkoxy, C₁-C₈-alkoxy-C₁-C₈-alkoxy, C₁-C₈-alkoxy-C₁-C₈-alkyl, C₁-C₈-alkoxycarbonylamino, C₁-C₈-alkanoylamino, C₁-C₈-alkylamino, N,N-di-C₁-C₈-alkylamino, aryl-C₀-C₄-alkyl, aryloxy-C₀-C₄-alkyl, aryl-C₀-C₄-alkyl-C₁-C₈₋alkoxy, aryloxy-C₀-C₄-alkyl-C₁-C₈-alkoxy, heterocyclyl-C₀-C₄-alkyl, heterocyclyloxy-C₀-C₄₋alkyl, heterocyclyl-C₀-C₄-alkyl-C₁-C₈-alkoxy or heterocyclyloxy-C₀-C₄-alkyl-C₁-C₈-alkoxy;
R₃ is hydrogen, C₁-C₈-alkyl, C₁-C₈-alkoxycarbonyl or C₁-C₈-alkanoyl;
R₄ is hydrogen, C₁-C₈-alkyl, C₁-C₈-alkoxycarbonylor C₁-C₈-alkanoyl;
R₅ are each independently hydrogen or C₁-C₈-alkyl or, together with the carbon atom to which they are bonded, are a C₃-C₈-cycloalkylidene radical;
R₆ is one oxygen atom or two hydrogen atoms;
R is optionally substituted arylamino, N-aryl-N-((lower alkoxy)(lower alkyl))amino, N-aryl-N-aryl(lower alkyl)amino or heterocyclyl bonded via a ring nitrogen atom;
and salts thereof.

The asymmetric carbon atoms present in compounds of the formula (I) may have R -, S- or R,S-configurations. Accordingly, the compounds present may occur as isomer mixtures or as pure isomers, in particular as diastereoisomer mixtures, enantiomer pairs or pure enantiomers.

In the context of the restrictions specified for the substituents of the formula (I), the individual substituents are defined as follows :

Aryl, and aryl in arylamino, aryl-C₀-C₄-alkyl, aryl(lower alkyl), N-aryl-N-(lower alkoxy)(lower alkyl)amino, N-aryl-N-aryl(lower alkyl)amino, contains generally 1 - 14, preferably 6 - 10 carbon atoms, and is, for example, phenyl, indenyl, e.g. 2- or 4-indenyl, or naphthyl, e.g. 1-or 2-naphthyl. Preference is given to aryl having 6 - 10 carbon atoms, in particular phenyl or 1- or 2-naphthyl. The radicals mentioned may be unsubstituted or, for example, mono- or polysubstituted, for example mono- or disubstituted, by a lower alkyl, hydroxyl, lower alkoxy, oxo, carbamoyl(lower alkoxy), C₃-C₈-cycloalkyl, C₁-C₈-alkylsulphonyl, C₃-C₈-cycloalkylsulphonyl, aryl-C₀-C₈-alkylsulphonyl, heterocyclylsulphonyl, C₃-C₈-cycloalkyl-C₁-C₈-alkanoyl, aryl-C₁-C₈-alkanoyl, C₁-C₈-alkanoyl, C₁-C₈-alkoxycarbonyl, optionally N-mono- or N,N-di-C₁-C₈-alkylated carbamoyl-C₀-C₈-alkyl, (lower alkyl) carbamoyl(lower alkoxy), di(lower alkyl) carbamoyl(lower alkoxy), amino, (lower alkyl)- or di(lower alkyl)amino, carboxyl, (lower alkoxy)carbonyl, carbamoyl, sulphamoyl, (lower alkane)sulphonyl, halogen, trifluoromethyl, nitro, phenyl, 5- or 6-membered heterocyclyl containing as a heteroatom 1 nitrogen, sulphur or oxygen atom, 2 nitrogen atoms, 1 nitrogen atom and 1 sulphur atom, or 1 nitrogen atom and 1 oxygen atom, such as pyridyl, and/or by cyano, and the substituent may be present in any position, for example the o-, m- or p-position of the phenyl radicals, or in the 3- or 4-position of the 1- or 2-naphthyl radical, and a plurality of identical or different substituents may also be present.

Arylamino is, for example, anilino or 1- or 2-naphthylamino which are each unsubstituted or substituted in the phenyl or naphthyl moiety as specified above.

Heterocyclyl, and heterocyclyl in heterocyclyl-C₀-C₄-alkyl has, for example, from 5 to 7 ring atoms in the heterocyclyl ring and may contain one ring nitrogen atom and/or one further ring heteroatom selected from oxygen, sulphur and nitrogen, is, for example, furanyl, tetrahydrofuranyl, pyranyl, tetrahydropyranyl, thiazolyl, oxazolyl, pyridinyl or imidazolyl which are each unsubstituted or substituted by C₁-C₈-alkyl, halogen, oxo, oxide, cyano, trifluoromethyl, C₁-C₈-alkoxy, C₁-C₈-alkanoyl, C₁-C₈-alkoxycarbonyl, aryl or heterocyclyl.

Heterocyclyl-C₀-C₄-alkyl is, for example, pyridinyl, methylenepyridinyl or imidazolyl.

In the case of nitrogen heterocycles, the heterocyclyl radicals can be bonded either via the nitrogen or via a ring carbon.

Heterocycyl bonded via a ring nitrogen atom and having from 4 to 8 ring atoms has in particular from 5 to 7 ring atoms and may have 1 or 2 fused-on phenyl or cycloalkyl radicals, or else be present as a spiro compound. Examples include pyrrolidino, piperidino, piperazino, morpholino, thiomorpholino, indolin-1-yl, isoindolin-2-yl, 2,3-dihydrobenzimidazol-1 -yl, 1,2,3,4-tetrahydroquinol-1-yl, 1,2,3,4-tetrahydroisoquinol-2-yl, 1,2,3,4-tetrahydro-1,3-benzodiazin-1-yl or -3-yl, 1,2,3,4-tetrahydro-1,4-benzodiazin-1-yl, 3,4-dihydro-2H-1,4-benzoxazin-4-yl, 3,4-dihydro-2H-1,4-benzothiazin-4-yl, 3,4-dihydro-2H-1,3-benzothiazin-1-yl, 3,4,5,6,7,8-hexahydro-2H-1,4-benzoxazin-4-yl, 3,4,5,6,7,8-hexahydro-2H-1,4-benzothiazin-4-yl, 2,3,4,5-tetrahydro-1H-1-benz[6,7-b]azepin-1-yl and 5,6-dihydrophenanthridin-5-yl. Preference is given to benzofused 5- to 7-membered aza-, diaza-, azoxa- and azathiacycloalkenyl radicals bonded via a nitrogen atom, in particular indolin-1-yl, 1,2,3,4-tetrahydroquinolin-1-yl, 1,2,3,4-tetrahydroisoquinolin-2-yl, 1,2,3,4-tetrahydro-1,3-benzodiazin-1-yl, 1,2,3,4-tetrahydro-1,4-benzodiazin-1-yl, 3,4-dihydro-2H-1,4-benzoxazin-4-yl, 3,4-dihydro-2H-1,4-benzothiazin-4-yl, 3,4-dihydro-2H-1,3-benzothiazin-1-yl and 2,3,4,5-tetrahydro-1H-1-benz[6,7-b]azepin-1-yl. Further preferred for -NR₁R₂ are in particular pyrrolidino, piperidino, morpholino, 9-azabicyclo[3.3.1]non-9-yl, 1-azepan-1-yl, 2,8-diazaspiro[4.5]dec-8-yl, octahydroisoindol-2-yl, 4-azatricyclo[5.2.1.0^{2,6}]dec-4-yl, 3-azabicyclo[3.2.1]oct-3-yl, 3,7-diazabicyclo[3.3.1]non-3-yl, 3-azabicyclo[3.3.1]non-3-yl, 8-azabicyclo[3.2.1]oct-8-yl, 3-azabicyclo[3.2.2]non-3-yl and tetrahydro-1H-1-benz[6,7-b]azepin-1-yl.

The radicals mentioned may be unsubstituted or N-substituted and/or C-substituted, in which case in particular 1, 2 or 3 substituents may be present.

Examples of useful nitrogen substituents are, for example, lower alkyl, lower alkanoyl, (lower alkoxy)carbonyl, (lower alkane)sulphonyl, oxide, aryl or heteroaryl. Carbon substituents are, for example, lower alkyl, hydroxy(lower alkyl), (lower alkoxy)(lower alkyl), (lower alkenyl)oxy(lower alkyl), naphthoxy(lower alkyl), phenyloxy(lower alkyl), phenyl(lower alkoxy)(lower alkyl), (lower alkanoyl)oxy(lower alkyl), benzoyloxy(lower alkyl), (lower alkoxy)carbonyloxy(lower alkyl), phenyloxycarbonyloxy(lower alkyl), phenyl(lower alkoxy)carbonyloxy(lower alkyl), amino(lower alkyl), N-(lower alkyl)amino(lower alkyl), N,N-di(lower alkyl)amino(lower alkyl), carbamoyl(lower alkyl), (lower alkanoyl)amino(lower alkyl), benzoylamino(lower alkyl), (lower alkoxy)carbonylamino(lower alkyl), (lower alkoxy)-carbonyl(lower alkyl), (lower alkoxy)(lower alkoxy)(lower alkyl), (lower alkyl)thio(lower alkoxy)(lower alkyl), N-(lower alkoxy)imino(lower alkyl), cycloalkoxy(lower alkyl), cycloalkyl(lower alkoxy)(lower alkyl), lower alkenyl, (lower alkenyl)oxy, (lower alkoxy)(lower alkenyl), lower alkynyl, (lower alkynyl)oxy, lower alkanoyl, oxo, hydroxy, lower alkoxy, carbamoyl(lower alkoxy), N-(lower alkyl)carbamoyl(lower alkoxy), N-(lower alkyl) carbamoyloxy, N,N-di(lower alkyl)carbamoyloxy, (lower alkoxy)(lower alkoxy), (lower alkyl)thio(lower alkoxy), (lower alkanoyl)oxy, benzoyloxy, N-(lower alkyl)carbamoyl, amino, N-(lower alkyl)amino, N,N-di(lower alkyl)amino, (lower alkanoyl)amino, benzoylamino, cycloalkylcarbonylamino, cycloalkyl(lower alkanoyl)amino, (lower alkoxy)carbonyl(lower alkyl)amino, (lower alkenyl)oxycarbonylamino, (lower alkoxy)(lower alkoxy)carbonylamino, (lower alkoxy)(lower alkanoyl)amino, N-(lower alkyl)carbamoylamino, N,N-di(lower alkyl)-carbamoylamino, N-(lower alkanoyl)-N-(lower alkyl)amino, (lower alkoxy)carbonylamino, N-(lower alkoxy)carbonyl-N-(lower alkyl)amino, N,N-(lower alkylene)amino, N,N-(1-oxo(lower alkylene))amino, N,N-(1-oxo-2-oxa(lower alkylene))amino, carboxy, (lower alkoxy)carbonyl, phenyl(lower alkoxy)carbonyl, phenyloxycarbonyl, pyrrolidinylcarbonyl, piperidinylcarbonyl, piperazinylcarbonyl, morpholinylcarbonyl, thiomorpholinylcarbonyl, S,S-dioxothiomorpholin-4-ylcarbonyl, cyano, carbamoyl, N,N-di(lower alkyl)carbamoyl, N-(lower alkenyl)carbamoyl, N-cycloalkylcarbamoyl, N-cycloalkyl(lower alkyl)carbamoyl, N-hydroxy(lower alkyl)carbamoyl, N-(lower alkoxy)(lower alkyl)carbamoyl, N-carboxy(lower alkyl)carbamoyl, carbamoyl(lower alkyl)carbamoyl, (lower alkoxy)carbonyl(lower alkyl)carbamoyl, phenyl, dioxolan-2-yl, oxazol-2-yl, oxazolin-2-yl, oxazolidin-2-yl, nitro, sulphamoyl, (lower alkane)sulphonyl, phosphono, (lower alkane)phosphono, di(lower alkyl)phosphono, polyhalo(lower alkyl) and halogen.

Depending on the presence of asymmetric carbon atoms, the inventive compounds may be present in the form of isomer mixtures, especially as racemates, or in the form of pure isomers, especially of optical antipodes.

The compound groups mentioned below are not to be regarded as closed, but rather it is possible in a sensible manner to exchange parts of these compound groups with one another or with the definitions given above, or to omit parts, for example to replace general by more specific definitions.

Preference is given to ompounds of the formula (I) where R₆ is oxygen.

Particularly preferred R radicals are bicyclic radicals of the formula (la) 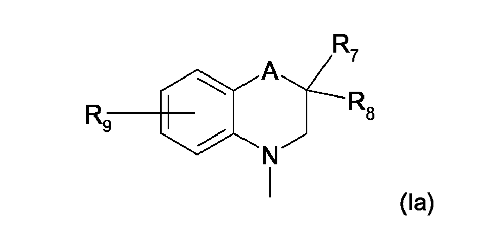 where A is a direct bond, methylene, dimethylene, imino, oxy or thio, R₇ is (lower alkoxy)(lower alkyl), (lower alkenyl)oxy(lower alkyl), (lower alkoxy)(lower alkoxy)(lower alkyl), (lower alkoxy)carbonylamino(lower alkyl), N-(lower alkoxy)imino(lower alkyl), phenyl, (lower alkoxy)carbonyl, cyano, carbamoyl, N-(lower alkyl)carbamoyl, N-((lower alkoxy)(lower alkyl))carbamoyl, (lower alkoxy), (lower alkoxy)(lower alkoxy), (lower alkanoyl)oxy, benzoyloxy, (lower alkanoyl)amino, (lower alkoxy)carbonylamino, 3- to 6-membered cycloalkylcarbonylamino, N-((lower alkoxy)(lower alkanoyl))amino, N-((lower alkyl)-carbamoyl)amino, N,N-(1-oxo(lower alkylene))amino, or N,N-(1-oxo-2-oxa(lower alkylene))amino, R₈ is hydrogen or lower alkyl and R₉ is hydrogen or halogen.

Above and below, "lower" radicals and compounds refer, for example, to those which have up to and including 8, preferably up to and including 4, carbon atoms.

C₁-C₆-alkylenedioxy is, for example, methylenedioxy or ethylenedioxy, but may also be 1,3-or 1,2-propylenedioxy.

Aryl-C₁-C₈-alkanoyl is one of the aryl radicals mentioned which is bonded to the rest of the compound via a C₁-C₈-alkanoyl group, for example phenylformyl, phenylacetyl, 3-phenylpropionyl, 2-phenyl-2-methylpropionyl or phenylpivaloyl.

Aryl-C₃-C₈-cycloalkanoyl is one of the aryl radicals mentioned which is bonded to the rest of the compound via a C₃-C₈-cycloalkanoyl group, for example 1-phenycyclobutanoyl.

Aryl(lower alkyl) is, for example, phenyl- or naphthyl(lower alkyl) which are each unsubstituted or substituted in the phenyl or naphthyl moiety as specified above.

Aryl-C₀-C₈-alkylsulphonyl is one of the aryl radicals mentioned which is bonded to the rest of the compound either via a sulphonyl group or via a C₁-C₈-alkylsulphonyl group, for example phenylsulphonyl, benzylsulphonyl or phenyldimethylenesulphonyl.

Optionally esterified carboxyl is, for example, carboxyl esterified with C₀-C₆-alkyl, such as carboxyl or C₁-C₆-alkoxycarbonyl.

Cycloalkoxy(lower alkyl) is, for example, C₃-C₈-cycloalkoxy-C₁-C₄-alkyl, such as cyclopropyloxy-C₁-C₄-alkyl, cyclopentyloxy-C₁-C₄-alkyl or cyclohexyloxy-C₁-C₄-alkyl, in particular cyclopropyloxymethyl.

Cycloalkoxy is, for example, C₃-C₈-cycloalkoxy, such as cyclopropyloxy, cyclopentyloxy or cyclohexyloxy, in particular cyclopropyloxy.

Cycloalkyl is, for example, 3- to 12-, in particular 3- to 6-membered cycloalkyl, such as cyclopropyl, cyclobutyl, cyclopentyl, cyclohexyl or adamantyl.

Cycloalkylidene is, for example, 3- to 8-, in particular 3- to 6-membered cycloalkylidene, such as cyclopropylidene, cyclobutylidene, cyclopentylidene or cyclohexylidene.

Cycloalkyl(lower alkyl) is, for example, 3- to 8-, in particular 3- to 6-membered cycloalkyl(lower alkyl), such as cyclopropyl-, cyclobutyl-, cyclopentyl- or cyclohexyl(lower alkyl).

N-aryl-N-(lower alkoxy)(lower alkyl)amino is, for example, N-phenyl- or N-naphthyl-N-(lower alkoxy)(lower alkyl)amino which are each unsubstituted or substituted in the phenyl or naphthyl moiety as specified above.

N-aryl-N-aryl(lower alkyl)amino is, for example, N-phenyl- or N-naphthyl-N-(phenyl(lower alkyl))amino which are each unsubstituted or substituted in the phenyl or naphthyl moiety as specified above.

Cycloalkyl(lower alkanoyl) is, for example C₃-C₈-cycloalkyl-C₁-C₄-alkanoyl, such as cyclopropyl-C₁-C₄-alkanoyl, cyclopentyl-C₁-C₄-alkanoyl or cyclohexyl-C₁-C₄-alkanoyl, in particular cyclopropylacetyl.

C₃-C₈-cycloalkylsulphonyl is, for example, cyclopentylsulphonyl, cyclohexylsulphonyl or cycloheptylsulphonyl, and also cyclopropylsulphonyl, cyclobutylsulphonyl or cyclooctylsulphonyl.

Halogen is, for example, fluorine, chlorine, bromine or iodine, preferably fluorine and chlorine.

Heterocyclyl-C₀-C₄-alkyl is one of the heterocyclyl radicals mentioned which is bonded to the rest of the compound either directly or via a C₁-C₄-alkyl group.

Heterocyclylsulphonyl is one of the heterocyclyl radicals mentioned which is bonded to the rest of the compound via a sulphonyl group.

Lower alkanoyl is, for example, C₁-C₈-alkanoyl, in particular C₁-C₄-alkanoyl, such as formyl, acetyl, propionyl, butyryl or pivaloyl. Lower alkanoyl R₃ is in particular formyl, acetyl, propionyl, butyryl, isobutyryl or pivaloyl.

(Lower alkanoyl)amino is, for example, C₁-C₈-alkanoylamino, in particular C₁-C₄₋alkanoylamino, such as acetylamino, propionylamino, butyrylamino or pivaloylamino.

(Lower alkanoyl)amino(lower alkyl) is, for example, C₁-C₈-alkanoylamino-C₁-C₄-alkyl, in particular C₁-C₄-alkanoylamino-C₁-C₄-alkyl, such as formylaminomethyl, acetylaminomethyl, propionylaminomethyl, butyrylaminomethyl, pivaloylaminomethyl, 2-formylaminoethyl, 2-acetylaminoethyl, 2-propionylaminomethyl, 2-butyrylaminoethyl or 2-pivaloylaminoethyl.

N-(lower alkanoyl)-N-(lower alkyl)amino is, for example, N-(C₁-C₈-alkanoyl)-N-(C₁-C₄₋alkyl)amino, in particular N-(C₁-C₄-alkanoyl)-N-(C₁-C₄-alkyl)amino, such as N-formyl-N-methylamino, N-acetyl-N-methylamino, N-propionyl-N-methylamino or N-butyryl-N-methylamino.

(Lower alkyl)sulphonyl is, for example, C₁-C₈-alkylsulphonyl, in particular C₁-C₄-alkylsulphonyl, such as methylsulphonyl, ethylsulphonyl, propylsulphonyl, prop-2-ylsulphonyl, butylsulphonyl, 2-methylpropylsulphonyl, but-2-ylsulphonyl or 2,2-dimethylethylsulphonyl.

Lower alkenyl is, for example, C₂-C₈-alkenyl, in particular C₃-C₅-alkenyl, such as allyl.

Lower alkynyl is, for example, C₃-C₈-alkynyl, in particular C₃-C₅-alkynyl, such as propargyl.

Lower alkoxy is, for example, C₁-C₈-alkoxy, in particular C₁-C₄-alkoxy, such as methoxy, ethoxy, propyloxy, isopropyloxy, butyloxy, isobutyloxy, sec-butyloxy or tert-butyloxy, but may also be a C₅-C₈-alkoxy group, such as a pentyloxy, hexyloxy or heptyloxy group.

(Lower alkoxy)carbonyl is, for example, C₁-C₈-alkoxycarbonyl, in particular C₁-C₄₋alkoxycarbonyl, such as methoxycarbonyl, ethoxycarbonyl, propyloxycarbonyl, isopropyloxycarbonyl, butyloxycarbonyl, isobutyloxycarbonyl, sec-butyloxycarbonyl or tert-butyloxycarbonyl.

(Lower alkoxy)carbonylamino is, for example, C₁-C₈-alkoxycarbonylamino, in particular C₁-C₄-alkoxycarbonylamino, such as methoxycarbonylamino, ethoxycarbonylamino, propyloxycarbonylamino, isopropyloxycarbonylamino or butyloxycarbonylamino.

(Lower alkoxy)(lower alkanoyl)amino is, for example, C₁-C₄-alkoxy-C₁-C₄-alkanoylamino, such as methoxy-C₁-C₄-alkanoylamino, ethoxy-C₁-C₄-alkanoylamino, propyloxy-C₁-C₄₋alkanoylamino, isopropyloxy-C₁-C₄-alkanoylamino or butyloxy-C₁-C₄-alkanoylamino, where C₁-C₄-alkanoyl is, for example, acetyl, propionyl or butyryl.

(Lower alkoxy)(lower alkoxy) is, for example, C₁-C₈-alkoxy-C₁-C₄-alkoxy, in particular C₁-C₄₋alkoxy-C₁-C₄-alkoxy, such as methoxy-C₁-C₄-alkoxy, ethoxy-C₁-C₄-alkoxy, propyloxy-C₁-C₄₋alkoxy, isopropyloxy-C₁-C₄-alkoxy, butyloxy-C₁-C₄-alkoxy, isobutyloxy-C₁-C₄-alkoxy, sec-butyloxy-C₁-C₄-alkoxy or tert-butyloxy-C₁-C₄-alkoxy, where C₁-C₄-alkoxy is, for example, methoxy, ethoxy, propyloxy or butyloxy, in particular methoxy- or ethoxymethoxy or 2-(methoxy)- or 2-(ethoxy)ethoxy.

(Lower alkoxy)(lower alkoxy)(lower alkyl) is, for example, C₁-C₈-alkoxy-C₁-C₄-alkoxy-C₁-C₄₋alkyl, in particular C₁-C₄-alkoxy-C₁-C₄-alkoxy-C₁-C₄-alkyl, such as methoxy-C₁-C₄-alkoxy-C₁₋C₄-alkyl, ethoxy-C₁-C₄-alkoxy-C₁-C₄-alkyl, propyloxy-C₁-C₄-alkoxy-C₁-C₄-alkyl, isopropyloxy-C₁-C₄-alkoxy-C₁-C₄-alkyl or butyloxy-C₁-C₄-alkoxy-C₁-C₄-alkyl, where C₁-C₄-alkoxy is, for example, methoxy, ethoxy, propyloxy or butyloxy and C₁-C₄-alkyl is, for example, methyl, ethyl, propyl or butyl, in particular 2-methoxyethoxymethyl.

(Lower alkoxy)(lower alkyl) is, for example, C₁-C₈-alkoxy-C₁-C₄-alkyl, in particular C₁-C₄₋alkoxy-C₁-C₄-alkyl, such as methoxy-C₁-C₄-alkyl, ethoxy-C₁-C₄-alkyl, propyloxy-C₁-C₄-alkyl, isopropyloxy-C₁-C₄-alkyl, butyloxy-C₁-C₄-alkyl, isobutyloxy-C₁-C₄-alkyl, sec-butyloxy-C₁-C₄₋alkyl or tert-butyloxy-C₁-C₄-alkyl, where C₁-C₄-alkyl is, for example, methyl, ethyl, propyl or butyl, in particular ethoxymethyl.

Lower alkyl is branched or unbranched and is, for example, C₁-C₈-alkyl, in particular C₁-C₄-alkyl, such as methyl, ethyl, n-propyl, isopropyl, n-butyl, isobutyl, sec-butyl, tert-butyl, n-pentyl, isopentyl, neopentyl, tert-pentyl, n-hexyl, isohexyl or n-heptyl.

N-(lower alkyl)amino is, for example, N-C₁-C₈-alkylamino, in particular C₁-C₄-alkylamino, such as methylamino, ethylamino, propylamino, butylamino, isobutylamino, sec-butylamino or tert-butylamino.

N,N-di(lower alkyl)amino is, for example, N,N-di-C₁-C₄-alkylamino, such as N,N-dimethylamino, N,N-diethylamino, N,N-dipropylamino, N-methyl-N-ethylamino or N-methyl-N-propylamino.

Optionally N-mono or N,N-di-C₁-C₈-alkylated carbamoyl-C₀-C₈-alkyl is, for example, carbamoyl, N-methylcarbamoyl, N-ethylcarbamoyl, N-propylcarbamoyl, N-butylcarbamoyl, N-methylcarbamoyl-C₁-C₈-alkyl, N-ethylcarbamoyl-C₁-C₈-alkyl, N-propylcarbamoyl-C₁-C₈₋alkyl, N-butylcarbamoyl-C₁-C₈-alkyl, N,N-di-C₁-C₄-alkylamino, such as N,N-dimethylcarbamoyl-C₁-C₈-alkyl, N,N-diethylcarbamoyl-C₁-C₈-alkyl, N,N-dipropylcarbamoyl-C₁-C₈-alkyl, N-methyl-N-ethylcarbamoyl-C₁-C₈-alkyl or N-methyl-N-propylcarbamoyl-C₁-C₈-alkyl, where C₁-C₈-alkyl is, for example, methyl or ethyl.

N-phenyl-N-(lower alkoxy)(lower alkyl)amino is, for example, N-phenyl-N-(C₁-C₄-alkoxy-C₁-C₄-alkyl)amino, such as N-phenyl-N-(methoxy-C₁-C₄-alkyl)amino, N-phenyl-N-(ethoxy-C₁-C₄-alkyl)amino, N-phenyl-N-(propyloxy-C₁-C₄-alkyl)amino, N-phenyl-N-(isopropyloxy-C₁-C₄-alkyl)amino or N-phenyl-N-(butyloxy-C₁-C₄-alkyl)amino, where C₁-C₄-alkyl is, for example, methyl, ethyl, propyl or butyl, in particular N-phenyl-N-(ethoxymethyl)amino.

N-phenyl-N-(lower alkyl)amino is, for example, N-phenyl-N-C₁-C₄-alkylamino, such as N-phenyl-N-methylamino, N-phenyl-N-ethylamino, N-phenyl-N-propylamino, N-phenyl-N-isopropylamino or N-phenyl-N-butylamino, in particular N-phenyl-N-methylamino.

N-phenyl-N-(phenyl(lower alkyl))amino is, for example, N-phenyl-N-(phenyl-C₁-C₄₋alkyl)amino, such as N-phenyl-N-benzylamino, N-phenyl-N-(2-phenylethyl)amino, N-phenyl-N-(3-phenylpropyl)amino or N-phenyl-N-(4-phenylbutyl)amino, in particular N-phenyl-N-(2-phenylethyl)amino.

Phenyl(lower alkanoyl) is, for example, phenyl-C₁-C₄-alkanoyl, where C₁-C₄-alkanoyl is, for example, acetyl, in particular phenylacetyl.

Polyhalo(lower alkyl) is, for example, di-, tri- or tetrahalo-C₁-C₄-alkyl, such as trifluoromethyl.

Pyridyl(lower alkyl) is, for example, pyridyl-C₁-C₄-alkyl, in particular pyrid-2-yl-C₁-C₄-alkyl, where C₁-C₄-alkyl is, for example, methyl, in particular pyrid-2-ylmethyl or 2-(pyrid-2-yl)ethyl.

Salts of compounds having salt-forming groups are in particular acid addition salts, salts with bases or, in the presence of a plurality of salt-forming groups, in some cases also mixed salts or internal salts. Salts are primarily the pharmaceutically usable or nontoxic salts of compounds of the formula I.

Such salts are formed, for example, from compounds of the formula I with an acidic group, for example a carboxyl or sulpho group, and are, for example, the salts thereof with suitable bases, such as nontoxic metal salts derived from metals of group la, Ib, IIa and IIb of the Periodic Table of the Elements, for example alkali metal, in particular lithium, sodium or potassium salts, alkaline earth metal salts, for example magnesium or calcium salts, and also zinc salts or ammonium salts, including those salts which are formed with organic amines, such as optionally hydroxy-substituted mono-, di- or trialkylamines, in particular mono-, di- or tri(lower alkyl)amines, or with quaternary ammonium bases, for example methyl-, ethyl-, diethyl- or triethylamine, mono-, bis- or tris(2-hydroxy(lower alkyl))amines, such as ethanol-, diethanol- or triethanolamine, tris(hydroxymethyl)methylamine or 2-hydroxy-tert-butylamine, N,N-di(lower alkyl)-N-(hydroxy(lower alkyl))amines, such as N,N-dimethyl-N-(2-hydroxyethyl)amine, or N-methyl-D-glucamine, or quaternary ammonium hydroxides, such as tetrabutylammonium hydroxide. The compounds of the formula I having a basic group, for example an amino group, may form acid addition salts, for example with suitable inorganic acids, e.g. hydrohalic acid such as hydrochloric acid, hydrobromic acid, sulphuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulphonic, sulpho or phosphonic acids or N-substituted sulphamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, and also amino acids, for example the α-amino acids mentioned above, and also methanesulphonic acid, ethanesulphonic acid, 2-hydroxyethanesulphonic acid, ethane-1,2-disulphonic acid, benzenesulphonic acid, 4-methylbenzenesulphonic acid, naphthalene-2-sulphonic acid, 2- or 3-phosphoglycerate, glucose 6-phosphate, N-cyclohexylsulphamic acid (with formation of cyclamates) or with other acidic organic compounds such as ascorbic acid. Compounds of the formula I with acidic and basic groups may also form internal salts.

For the isolation and purification, pharmaceutically unsuitable salts may also find use.

The invention relates, for example, to compounds of the formula I where
R₁ a) is hydrogen; or
is b) C₁-C₈-alkyl, C₃-C₈-cycloalkyl, C₁-C₈-alkanoyl, C₁-C₈-alkoxycarbonyl, aryl-C₀-C₄-alkyl or heterocyclyl-C₀-C₄-alkyl, which radicals may be substituted by 1 - 4 C₁-C₈-alkyl, halogen, cyano, oxide, oxo, trifluoromethyl, C₁-C₈-alkoxy, C₁-C₈-alkoxycarbonyl, aryl or heterocyclyl;
R₂ is a) C₁-C₈-alkyl, C₃-C₈-cycloalkyl, C₁-C₈-alkylsulphonyl, C₃-C₈-cycloalkylsulphonyl, aryl-C₀-C₈-alkylsulphonyl, heterocyclylsulphonyl, C₃-C₈-cycloalkyl-C₁-C₈-alkanoyl, aryl-C₁-C₈₋alkanoyl, aryl-C₃-C₈-cycloalkanoyl, C₁-C₈-alkanoyl, C₁-C₈-alkoxycarbonyl, optionally N-mono- or N,N-di-C₁-C₈-alkylated carbamoyl-C₀-C₈-alkyl, aryl-C₀-C₄-alkyl or heterocyclyl-C₀-C₄-alkyl, which radicals may be substituted by 1-4 C₁-C₈-alkyl, C₃-C₈-cycloalkyl, C₃-C₈-cycloalkoxy, amino, C₁₋₆-alkylamino, di-C₁₋₆-alkylamino, C₀-C₆-alkylcarbonylamino, C₁-C₆-alkoxycarbonylamino, halogen, oxo, cyano, hydroxyl, oxide, trifluoromethyl, C₁-C₈-alkoxy, optionally N-mono- or N,N-di-C₁-C₈-alkylated carbamoyl-C₀-C₈-alkyl, optionally esterified carboxyl, C₁₋₆₋alkylenedioxy, aryl or heterocyclyl; or
is b) together with R₁ and the nitrogen atom to which they are bonded, a saturated or partly unsaturated 4 - 8-membered heterocyclic ring which may contain an additional nitrogen, oxygen or sulphur atom or a -SO- or -SO2- group, in which case the additional nitrogen atom may optionally be substituted by C₁-C₈-alkyl, C₁-C₈-alkanoyl, C₁-C₈-alkoxycarbonyl, aryl or heteroaryl radicals, and this heterocyclic ring may be part of a bicyclic or tricyclic ring system having a total of up to 16 members and the second ring may also contain a nitrogen, oxygen or sulphur atom or a -SO- or -SO2- group, and the nitrogen atom in the second ring may optionally be substituted by C₁-C₈-alkyl, C₁-C₈-alkanoyl, C₁-C₈-alkoxycarbonyl, aryl or heterocyclyl radicals, and all ring systems mentioned may be substituted by 1 - 4 C₁-C₈-alkyl, halogen, hydroxyl, cyano, oxide, oxo, trifluoromethyl, C₁-C₈-alkoxy, C₁-C₈-alkoxy-C₁-C₈₋alkoxy, C₁-C₈-alkoxycarbonylamino, C₀-C₈-alkylcarbonylamino, C₁-C₈-alkylamino, N,N-di-C₁₋C₈-alkylamino, aryl-C₀-C₄-alkyl, aryloxy-C₀-C₄-alkyl, aryl-C₀-C₄-alkyl-C₁-C₈-alkoxy, aryloxy-C₀₋C₄-alkyl-C₁-C₈-alkoxy, heterocyclyl-C₀-C₄-alkyl, heterocyclyloxy-C₀-C₄-alkyl, heterocyclyl-C₀₋C₄-a)ky)-C₁-C₈-alkoxy or heterocyclyloxy-C₀-C₄-alkyl-C₁-C₈-alkoxy;
R₃ is hydrogen, C₁-C₈-alkyl, C₁-C₈-alkoxycarbonyl or C₁-C₈-alkanoyl;
R₄ is hydrogen, C₁-C₈-alkyl, C₁-C₈-alkoxycarbonylor C₁-C₈-alkanoyl;
R₅ are each independently hydrogen or C₁-C₈-alkyl,
R₆ is oxygen,
R is arylamino, N-aryl-N-((lower alkoxy)(lower alkyl))amino, N-aryl-N-aryl(lower alkyl)amino or heterocyclyl bonded via a ring nitrogen atom, in which case the heterocyclyl mentioned, apart from the ring nitrogen atom via which it is bonded, may contain further ring heteroatoms selected from oxygen, nitrogen, nitrogen substituted by lower alkyl, lower alkanoyl, (lower alkane)sulphonyl or (lower alkoxy)carbonyl, sulphur, and sulphur bonded to 1 or 2 oxygen atoms,
and salts thereof.

The invention relates primarily to compounds of the formula I where R is unsubstituted or anilino or naphthylamino which are each unsubstituted or substituted in the phenyl or naphthyl moiety as specified below, N-phenyl- or N-naphthyl-N-(lower alkoxy)(lower alkyl)amino which are each unsubstituted or substituted in the phenyl or naphthyl moiety as specified, N-phenyl- or N-naphthyl-N-(lower alkyl)amino which are each unsubstituted or substituted in the phenyl or naphthyl moiety as specified, or 5- to 8-membered heterocyclyl which is bonded via a ring nitrogen atom and is optionally fused with 1 or 2 fused-on phenyl or cycloalkyl radicals, and which may contain 1 or 2 further ring heteroatoms selected from oxygen, nitrogen and optionally oxidized sulphur, where phenyl, naphthyl and phenyl or naphthyl radicals as a constituent of napthylamino, N-phenyl- or N-naphthyl-N-(lower alkoxy)(lower alkyl)amino, N-phenyl- or N-naphthyl-N-(lower alkyl)amino, may be mono- or polysubstituted, for example mono- or disubstituted, by lower alkyl, hydroxy, lower alkoxy, carbamoyl(lower alkoxy), N-(lower alkyl)carbamoyl(lower alkoxy), N-(lower alkyl)carbamoyl, N,N-di(lower alkyl)carbamoyl(lower alkoxy), amino, N-(lower alkyl)- or N,N-di(lower alkyl)amino, carboxy, (lower alkoxy)carbonyl, carbamoyl, sulphamoyl, (lower alkane)sulphonyl, halogen, nitro, phenyl, 5- or 6-membered heteroaryl containing as a heteroatom 1 nitrogen, sulphur or oxygen atom, 2 nitrogen atoms, 1 nitrogen atom and 1 sulphur atom, or 1 nitrogen and 1 oxygen atom, such as pyridyl, and/or by cyano, and R radicals may be N-substituted by lower alkyl, lower alkanoyl, (lower alkoxy)carbonyl, or (lower alkane)sulphonyl, S-mono- or S,S-disubstituted by oxy, and/or mono- or di-C-substituted by lower alkyl, hydroxy(lower alkyl), (lower alkoxy)(lower alkyl), (lower alkenyl)oxy(lower alkyl), naphthoxy(lower alkyl), phenyloxy(lower alkyl), phenyl(lower alkoxy)(lower alkyl), (lower alkanoyl)oxy(lower alkyl), benzoyloxy(lower alkyl), (lower alkoxy)carbonyloxy(lower alkyl), phenyloxycarbonyloxy(lower alkyl), phenyl(lower alkoxy)carbonyloxy(lower alkyl), amino(lower alkyl), N-(lower alkyl)amino(lower alkyl), N,N-di(lower alkyl)amino(lower alkyl), carbamoyl(lower alkyl), (lower alkanoyl)amino(lower alkyl), benzoylamino(lower alkyl), (lower alkoxy)carbonylamino(lower alkyl), (lower alkoxy)carbonyl(lower alkyl), (lower alkoxy)(lower alkoxy)(lower alkyl), (lower alkyl)thio(lower alkoxy)(lower alkyl), N-(lower alkoxy)imino(lower alkyl), cycloalkoxy(lower alkyl), cycloalkyl(lower alkoxy)(lower alkyl), lower alkenyl, (lower alkenyl)oxy, (lower alkoxy)(lower alkenyl), lower alkynyl, (lower alkynyl)oxy, lower alkanoyl, oxo, hydroxyl, lower alkoxy, carbamoyl(lower alkoxy), N-(lower alkyl)carbamoyl(lower alkoxy), N-(lower alkyl)carbamoyloxy, N,N-di(lower alkyl)carbamoyloxy, (lower alkoxy)(lower alkoxy), (lower alkyl)thio(lower alkoxy), (lower alkanoyl)oxy, benzoyloxy, N-(lower alkyl)carbamoyl, amino, N-(lower alkyl)amino, N,N-di(lower alkyl)amino, (lower alkanoyl)amino, benzoylamino, cycloalkylcarbonylamino, cycloalkyl(lower alkanoyl)amino, (lower alkoxy)carbonyl(lower alkyl)amino, (lower alkenyl)oxycarbonylamino, (lower alkoxy)(lower alkoxy)carbonylamino, (lower alkoxy)(lower alkanoyl)amino, N-(lower alkyl)carbamoylamino, N,N-di(lower alkyl)carbamoylamino, N-(lower alkanoyl)-N-(lower alkyl)amino, (lower alkoxy)carbonylamino, N-(lower alkoxy)carbonyl-N-(lower alkyl)amino, N,N-(lower alkylene)amino, N,N-(1-oxo(lower alkylene))amino, N,N-(1-oxo-2-oxa(lower alkylene))amino, carboxyl, (lower alkoxy)carbonyl, phenyl(lower alkoxy)carbonyl, phenyloxycarbonyl, pyrrolidinylcarbonyl, piperidinylcarbonyl, piperazinylcarbonyl, morpholinylcarbonyl, thiomorpholinylcarbonyl, S,S-dioxothiomorpholin-4-ylcarbonyl, cyano, carbamoyl, N,N-di(lower alkyl)carbamoyl, N-(lower alkenyl)carbamoyl, N-cycloalkylcarbamoyl, N-cycloalkyl(lower alkyl)carbamoyl, N-hydroxy(lower alkyl)carbamoyl, N-(lower alkoxy)(lower alkyl)carbamoyl, N-carboxy(lower alkyl)carbamoyl, carbamoyl(lower alkyl)carbamoyl, (lower alkoxy)carbonyl(lower alkyl)carbamoyl, phenyl, dioxolan-2-yl, oxazol-2-yl, oxazolin-2-yl, oxazolidin-2-yl, nitro, sulphamoyl, (lower alkane)sulphonyl, phosphono, (lower alkane)phosphono, di(lower alkyl)phosphono and/or halogen.

The invention relates in particular to compounds of the formula I where R is an anilino, naphthylamino, N-phenyl-N-(C₁-C₄-alkoxy-C₁-C₄-alkyl)amino such as N-phenyl-N-(ethoxymethyl)amino, or N-phenyl-N-(phenyl-C₁-C₄-alkyl)amino such as N-phenyl-N-(2-phenylethyl)amino which are each unsubstituted, or mono- or disubstituted in the phenyl or naphthyl moiety by C₁-C₄-alkoxy such as methoxy, C₁-C₄-alkoxycarbonyl such as methoxy-, ethoxy-, isopropyloxy- or tert-butyloxycarbonyl, carbamoyl-C₁-C₄-alkoxy such as carbamoylmethoxy, C₁-C₄-alkanoylamino-C₁-C₄-alkyl such as formylaminomethyl, C₁-C₄₋alkoxycarbonylamino-C₁-C₄-alkyl such as methoxycarbonylaminomethyl, halogen and/or optionally N-oxidized pyridyl such as pyrid-3-yl or 1-oxidopyrid-3-yl, or is pyrrolidino, piperidino, piperazino, morpholino or thiomorpholino, indolin-1-yl, isoindolin-2-yl, 2,3-dihydrobenzimidazol-1-yl, 1,2,3,4-tetrahydroquinol-1-yl, 1,2,3,4-tetrahydroisoquinol-2-yl, 1,2,3,4-tetrahydro-1,3-benzodiazin-1-yl, 1,2,3,4-tetrahydro-1,4-benzodiazin-1-yl, 3,4-dihydro-2H-1,4-benzoxazin-4-yl, optionally S,S-dioxidized 3,4-dihydro-2H-1,3-benzothiazin-1-yl, 3,4,5,6,7,8-hexahydro-2H-1,4-benzoxazin-4-yl, 3,4,5,6,7,8-hexahydro-2H-1,4-benzothiazin-4-yl, 2,3,4,5-tetrahydro-1H-1-benzo[6,7-b]azepin-1-yl or 5,6-dihydrophenanthridin-5-yl which are each unsubstituted or mono-, di- or trisubstituted by C₁-C₄-alkyl such as methyl, hydroxy-C₁-C₄-alkyl such as hydroxymethyl, C₁-C₄-alkoxy-C₁-C₄-alkyl such as methoxymethyl or propyloxymethyl, C₃-C₅-alkenyloxy-C₁-C₄-alkyl such as allyloxymethyl, C₁-C₄-alkoxy-C₁-C₄₋alkoxy-C₁-C₄-alkyl such as methoxymethoxymethyl or 2-methoxyethoxymethyl, C₁-C₄₋alkoxycarbonylamino-C₁-C₄-alkyl such as methoxy- or ethoxycarbonylaminomethyl, C₁-C₄₋alkoxyimino-C₁-C₄-alkyl such as methoxyiminomethyl, ethoxyiminomethyl or propoxyiminomethyl, carboxyl, C₁-C₄-alkoxycarbonyl such as methoxy-, ethoxy-, isopropyloxy- or tert-butyloxycarbonyl, cyano, carbamoyl, N-C₁-C₈-alkylcarbamoyl such as N-methyl- or N-butylcarbamoyl, N,N-di-C₁-C₄-alkylcarbamoyl such as N,N-dimethylcarbamoyl, C₁-C₄-alkoxy-C₁-C₄-alkylcarbamoyl such as N-(2-propyloxyethyl)carbamoyl, N-carboxy-C₁-C₄-alkylcarbamoyl such as N-carboxymethylcarbamoyl, morpholinocarbonyl, C₁-C₄-alkoxy such as propyloxy, C₁-C₄-alkoxy-C₁-C₄-alkoxy such as methoxymethoxy or 2-methoxyethoxy, C₁-C₄-alkanoyloxy such as acetoxy or benzoyloxy, C₁-C₄-alkanoylamino, such as acetylamino, C₁-C₄-alkoxycarbonylamino such as methoxycarbonylamino, 3- to 6-membered cycloalkylcarbonylamino such as cyclopropylcarbonylamino, N-C₁-C₄-alkoxy-C₁-C₄-alkanoylamino such as methoxyacetylamino, N-C₁-C₄-alkylcarbamoylamino such as methylcarbamoylamino, or 5- or 6-membered N,N-(1-oxo(lower alkylene))amino or N,N-(1-oxo-2-oxa(lower alkylene))amino such as 2-oxopyrrolidin-1-yl or 2-oxooxazolidin-3-yl, C₁-C₈-alkanoyl such as acetyl, oxo, nitro, C₁-C₄-alkanesulphonyl such as methane- or ethanesulphonyl, and/or halogen.

The invention relates in particular to compounds of the formula I where R is a group of the formula 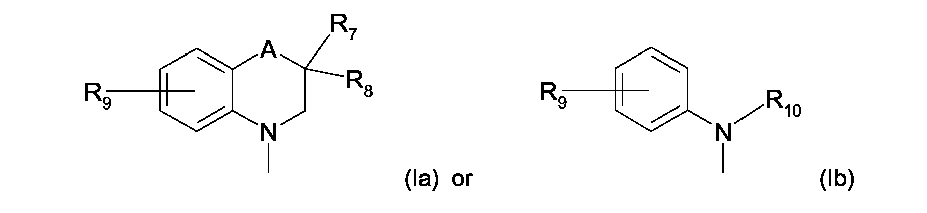 in which
A is a direct bond, methylene, dimethylene, imino, oxy or thio,
R₇ is C₁-C₄-alkoxy-C₁-C₄-alkyl, such as methoxy- or propyloxymethyl, C₃-C₅-alkenyloxy-C₁₋C₄-alkyl, such as allyloxymethyl, C₁-C₄-alkoxy-C₁-C₄-alkoxy-C₁-C₄-alkyl, such as methoxymethoxymethyl or 2-methoxyethoxymethyl, C₁-C₄-alkoxycarbonylamino-C₁-C₄-alkyl, such as methoxy- or ethoxycarbonylaminomethyl, C₁-C₄-alkoxyimino-C₁-C₄-alkyl, such as methoxyiminomethyl, phenyl, C₁-C₄-alkoxycarbonyl, such as methoxycarbonyl, ethoxycarbonyl or isopropyloxycarbonyl, cyano, carbamoyl, N-C₁-C₄-alkylcarbamoyl, such as N-methylcarbamoyl, N-ethylcarbamoyl or N-butylcarbamoyl, C₁-C₄-alkoxy-C₁-C₄₋alkylcarbamoyl, such as N-(2-methoxyethyl)carbamoyl, C₁-C₄-alkoxy such as propyloxy, C₁-C₄-alkoxy-C₁-C₄-alkoxy such as methoxymethoxy or 2-methoxyethoxy, C₁-C₈-alkanoyloxy such as acetoxy, benzoyloxy, N-C₁-C₄-alkylcarbamoylamino, such as N-methylcarbamoylamino, C₁-C₄-alkanoylamino, such as acetylamino, C₁-C₄-alkoxycarbonylamino, such as methoxycarbonylamino, 3- to 6-membered cycloalkylcarbonylamino, such as cyclopropylcarbonylamino, C₁-C₄-alkoxy-C₁-C₄-alkanoylamino, such as methoxyacetylamino, or 5- or 6-membered N,N-(1-oxo(lower alkylene))amino or N,N-(1-oxo-2-oxa(lower alkylene))amino, such as 2-oxopyrrolidin-1-yl or 2-oxooxazolidin-3-yl, N-C₁-C₄-alkylcarbamoylamino, such as methylcarbamoylamino,
R₈ is hydrogen, but may also be C₁-C₄-alkyl such as methyl,
R₉ is hydrogen or halogen and
R₁₀ is C₁-C₄-alkoxy-C₁-C₄-alkyl, such as methoxy-C₁-C₄-alkyl, ethoxy-C₁-C₄-alkyl, propyloxy-C₁-C₄-alkyl, isopropyloxy-C₁-C₄-alkyl, butyloxy-C₁-C₄-alkyl, isobutyloxy-C₁-C₄-alkyl,
sec-butyloxy-C₁-C₄-alkyl or tert-butyloxy-C₁-C₄-alkyl, where C₁-C₄-alkyl is, for example, ethyl, propyl or butyl, and is in particular 3-methoxypropyl.

Particularly effective in each case are those compounds of the formula I with the stereochemistry (in each case "S") of the main chain shown in the formula

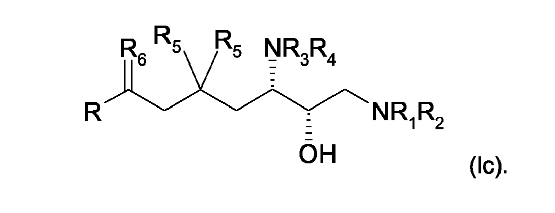

The invention relates in each case preferentially to the stereoisomers of compounds of the formula I with the stereochemistry of the main chain shown in the formula Ic, where the variables are each defined as follows, and salts thereof, in particular pharmaceutically usable salts thereof.

The invention relates specifically to the compounds of the formula I specified in the examples and to salts thereof, in particular to pharmaceutically usable salts thereof.

The invention relates primarily to compounds of the formula 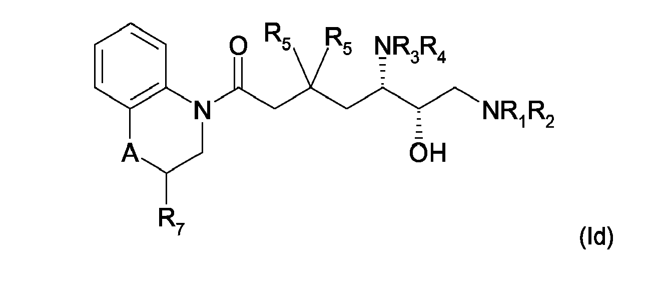 where
A is methylene, oxy or thio,
R₁ is a) hydrogen; or
is b) C₁-C₈-alkyl or C₃-C₈-cycloalkyl;
R₂ is a) C₁-C₈-alkyl, C₃-C₈-cycloalkyl, C₁-C₈-alkanoyl, heterocyclyl-C₁-C₈-alkanoyl, C₃-C₁₂₋cycloalkyl-C₁-C₈-alkanoyl or aryl-C₁-C₈-alkanoyl, which radicals may be substituted by 1 - 4 C₁-C₈-alkyl, C₃-C₈-cycloalkyl, C₃-C₈-cycloalkoxy, C₁₋₆-alkylamino, cyano, halogen, hydroxyl, oxide, C₀-C₆-alkylcarbonylamino, C₁-C₈-alkoxy, oxo, trifluoromethyl or aryl; or
b) together with R₁ and the nitrogen atom to which they are bonded, is a saturated or partly unsaturated, 4 - 8-membered heterocyclic ring which may contain an additional nitrogen or oxygen atom, in which case the additional nitrogen atom may optionally be substituted by C₁-C₈-alkyl or C₁-C₈-alkanoyl, and this heterocyclic ring may be part of a bicyclic or tricyclic ring system having a total of up to 16 members, and the second ring may also contain a nitrogen or oxygen atom, in which case the nitrogen atom of the second ring may optionally be substituted by C₁-C₈-alkyl or C₁-C₈-alkanoyl, and all ring systems mentioned may be substituted by 1 - 4 C₁-C₈-alkyl, hydroxyl, cyano, oxide, oxo, C₁-C₈-alkoxy, C₁-C₈-alkoxy-C₁₋C₈-alkoxy, C₀-C₈-alkylcarbonylamino, C₁-C₈-alkoxycarbonylamino or aryloxy-C₀-C₄-alkyl-C₁₋C₈-alkoxy;
R₃ is hydrogen or ―(C=O)-C₁-C₄-alkyl;
R₄ is hydrogen;
R₅ are each independently C₁-C₄-alkyl, such as methyl,
R₇ is C₁-C₄-alkoxycarbonylamino such as methoxycarbonylamino, ethoxycarbonylamino, propyloxycarbonylamino, isopropyloxycarbonylamino or butyloxycarbonylamino, C₁-C₄₋alkoxy-C₁-C₄-alkoxy-C₁-C₄-alkyl, such as methoxy-C₁-C₄-alkoxy-C₁-C₄-alkyl, ethoxy-C₁-C₄₋alkoxy-C₁-C₄-alkyl, propyloxy-C₁-C₄-alkoxy-C₁-C₄-alkyl, isopropyloxy-C₁-C₄-alkoxy-C₁-C₄-alkyl or butyloxy-C₁-C₄-alkoxy-C₁-C₄-alkyl, where C₁-C₄-alkoxy is, for example, methoxy, ethoxy, propyloxy or butyloxy, and C₁-C₄-alkyl is, for example, methyl, ethyl, propyl or butyl, in particular methoxymethoxymethyl, 2-methoxyethoxymethyl or 3-methoxypropyloxymethyl, C₁-C₄-alkoxy-C₁-C₄-alkyl, such as methoxy-C₁-C₄-alkyl, ethoxy-C₁-C₄-alkyl, propyloxy-C₁-C₄₋alkyl, isopropyloxy-C₁-C₄-alkyl, butyloxy-C₁-C₄-alkyl, isobutyloxy-C₁-C₄-alkyl, sec-butyloxy-C₁-C₄-alkyl or tert-butyloxy-C₁-C₄-alkyl, where C₁-C₄-alkyl is, for example, methyl, ethyl, propyl or butyl, in particular ethoxymethyl or 2-methoxyethyl, or N-C₁-C₄-alkylcarbamoyl, such as N-methylcarbamoyl, N-ethylcarbamoyl, N-propylcarbamoyl or N-butylcarbamoyl, and salts thereof, in particular the pharmaceutically usable salts thereof.

Salts obtained may be converted to other salts in a manner known per se, acid addition salts, for example, by treating with a suitable metal salt such as a sodium, barium or silver salt, of another acid in a suitable solvent in which an inorganic salt which forms is insoluble and thus separates out of the reaction equilibrium, and base salts by release of the free acid and salt reformation.

The compounds of the formula I, including their salts, may also be obtained in the form of hydrates or include the solvent used for the crystallization.

Stereoisomer mixtures, i.e. mixtures of diastereomers and/or enantiomers, for example racemic mixtures, may be separated into the corresponding isomers in a manner known per se by suitable separation processes. For instance, diastereomer mixtures may be separated into the individual diastereomers by fractional crystallization, chromatography, solvent partition, etc. After conversion of the optical antipodes to diastereomers, for example by reacting with optically active compounds, e.g. optically active acids or bases, racemates may be separated from one another by chromatography on column materials laden with optically active compounds or by enzymatic methods, for example by selective conversion of only one of the two enantiomers. This separation may be effected either at the stage of one of the starting materials or on the compounds of the formula I. It is possible for the configuration at individual chiral centres in a compound of the formula I to be inverted selectively.

For example, the configuration of asymmetric carbon atoms which bear nucleophilic substituents, such as amino or hydroxyl, may be inverted by second-order nucleophilic substitution, if appropriate after conversion of the bonded nucleophilic substituent to a suitable nucleofugic leaving group and reaction with a reagent which introduces the original substituents, or the configuration at carbon atoms having hydroxyl groups can be inverted by oxidation and reduction, analogously to the process in the European patent application EP-A-0 236 734.

Also advantageous is the reactive functional modification of the hydroxyl group and subsequent replacement thereof by hydroxyl with inversion of configuration. To this end, the amino and hydroxyl group drawn in formula I are bridged by a bivalent group, in particular carbonyl, to obtain a compound which can be cleaved again on treatment with thionyl chloride with inversion of configuration.

The compounds of the formula (I) may be prepared in an analogous manner to preparation processes known from the literature. The starting materials for carrying out the preparation processes are described, for example, in EP 0702004. The inventive compounds of the formula (I) and salts of such compounds having at least one salt-forming group are obtained by processes known per se, as also described on pages 17 to 20 of W02005/070870 which are herewith incorporated

The compounds of the formula (I) may also be prepared in optically pure form. The separation into antipodes may be effected by methods known per se, either preferably at a synthetically early stage by salt formation with an optically active acid, for example (+)- or (-)-mandelic acid and separation of the diastereomeric salts by fractional crystallization, or preferably at a rather later stage by derivatization with a chiral auxiliary building block, for example (+)- or (-)-camphanoyl chloride, and separation of the diastereomeric products by chromatography and/or crystallization and subsequent cleavage of the bond to the chiral auxiliary. To determine the absolute configuration of the piperidine present, the pure diastereomeric salts and derivatives may be analysed with common spectroscopic methods, of which X-ray spectroscopy on single crystals constitutes a particularly suitable method.

Prodrug derivatives of the compounds described in the present context are derivatives thereof which, on *in vivo* application, release the original compound by a chemical or physiological process. A prodrug may be converted to the original compound, for example, when a physiological pH is attained or by enzymatic conversion. Prodrug derivatives may, for example, be esters of freely available carboxylic acids, S- and O-acyl derivatives of thiols, alcohols or phenols, and the acyl group is as defined in the present context. Preference is given to pharmaceutically usable ester derivatives which are converted by solvolysis in physiological medium to the original carboxylic acid, for example lower alkyl esters, cycloalkyl esters, lower alkenyl esters, benzyl esters, mono- or disubstituted lower alkyl esters such as lower ω-(amino, mono- or dialkylamino, carboxyl, lower alkoxycarbonyl)-alkyl esters or such as lower α-(alkanoyloxy, alkoxycarbonyl or dialkylaminocarbonyl)-alkyl esters; as such, pivaloyloxymethyl esters and similar esters are utilized in a conventional manner.

Owing to the close relationship between a free compound, a prodrug derivative and a salt compound, a certain compound in this invention also encompasses its prodrug derivative and salt form, where this is possible and appropriate.

The compounds of the formula (I) also include those compounds in which one or more atoms are replaced by their stable, non-radioactive isotopes; for example, a hydrogen atom by deuterium.

The compounds of formula (I), (Ic) and (Id), respectively, and their pharmaceutically useful salts reveal inhibitory activities on the enzymes beta-secretase, cathepsin D, plasmepsin II and/or HIV-protease.
The activitiy of inhibitors of beta-secretase, cathepsin D, plasmepsin II and/or HIV protease can be assessed experimentally with following *in vitro* assays.

The protease inhibitory activity of compounds can be tested with an assay kit using the fluorescence resonance energy transfer (FRET) technology and a recombinant i.e. baculovirus expressed enzyme preparation. The FRET is used to monitor the cleavage of the peptide substrate. The principle of the assay is as follows relies on a measurable energy difference, quantitatively depending on the presence of a peptide sequence. The peptide substrate is synthesized with two terminal fluorophores, a fluorescent donor and quenching acceptor. The distance between these two groups is selected so that upon light excitation, the donor fluorescence energy is significantly quenched by the acceptor through resonance energy transfer. Upon cleavage by the protease, the fluorophore is separated from the quenching group, restoring the fluorescence yield of the donor. Thus a weakly fluorescent peptide substrate becomes highly fluorescent upon enzymatic cleavage; the increase in fluorescence is linearly related to the rate of proteolysis.

The FRET assay was performed in white polysorp plates. The assay buffer consisted of 50 mM sodium acetate pH 5, 392 mM sodium chloride, 12.5% glycerol and 0.1% BSA. The incubates per well were composed of 160 ul buffer, 10 ul inhibitor in DMSO, 10 ul peptide substrate in DMSO and 20 ul enzyme-solution. The inhibitors are tested in a concentration range of 1 pM to 1 mM. The fluorescently marked donor and acceptor peptide substrates are generated by solid phase peptide synthesis (Applied Biosystems). The beta-secretase peptide substrate Rh-Glu-Val-Asn-Leu-Asp-Ala-Glu-Phe-Lys-Quencher is obtained from Invitrogen, Carlsbad, CA, USA. The cathepsin D peptide substrate of the sequence DABCYL-Pro-Thr-Glu-Phe-Phe-Arg-Leu-OXL, the plasmepsin peptide substrate of the sequence DABCYL-Glu-Arg-Nle-Phe-Leu-Ser-Phe-Pro-OXL and the HIV protease peptide substrate of the sequence DABCYL-His-Lys-Ala-Arg-Val-Leu-Tyr-Glu-Ala-Nle-Ser-EDANS are all obtained from AnaSpec Inc, San Jose, CA, USA. The recombinantly expressed enzyme preparations are added in various amounts to the assay systems eg the beta-sectrase concentration is 1 unit/ml incubation volume, the cathepsin D concentration is 100 ng/ml, the HIV protease concentration is 500 ng/ml and the plasmepsin II concentration is 50 ng/ml. The reaction is started upon addition of the enzyme solution. The incubation occurs at 37°C over 30-120 min ie specifically the beta-secretase incubation lasts 60 min, the cathepsin D incubation 120 min, the plasmepsin II incubation 40 min and the HIV protease incubation 40 min. The reactions are stopped by the addition of 20 µl of a 1.0 M Tris Base solution. The enzymatic substrate to product conversion is assessed by fluorescence measurements at 460 nm wave length.

### In vitro enzyme inhibitory activities

The compounds of the present invention revealed structure-dependent and enzyme-specific inhibitory activities. The inhibitory activities were measured as IC50 values. Thus the beta-secretase inhibitory activity ranged between 1 pM and 1 mM; the values for cathepsin D ranged between 1 pM und 1 mM, for plasmepsin II between 1 pM und 1 mM and for HIV-protease between 1 pM und 1 mM.

The compounds of the formula (I) and the pharmaceutically usable salts thereof may find use as medicines, for example in the form of pharmaceutical preparations. The pharmaceutical preparations may be administered enterally, such as orally, for example in the form of tablets, coated tablets, sugar-coated tablets, hard and soft gelatine capsules, solutions, emulsions or suspensions, nasally, for example in the form of nasal sprays, rectally, for example in the form of suppositories, or transdermally, for example in the form of ointments or patches. The administration may also be parenteral, such as intramuscular or intravenous, for example in the form of injection solutions.

To prepare tablets, coated tablets, sugar-coated tablets and hard gelatine capsules, the compounds of the formula (I) and pharmaceutically usable salts thereof may be processed with pharmaceutically inert, inorganic or organic excipients. Such excipients used, for example for tablets, coated tablets and hard gelatine capsules, may be lactose, corn starch, or derivatives thereof, talc, stearic acid or salts thereof etc.

Suitable excipients for soft gelatine capsules are, for example, vegetable oils, waxes, fats, semisolid and liquid polyols, etc.

Suitable excipients for preparing solutions and syrups are, for example, water, polyols, sucrose, invert sugar, glucose, etc.

Suitable excipients for injection solutions are, for example, water, alcohols, polyols, glycerol, vegetable oils, bile acids, lecithin, etc.

Suitable excipients for suppositories are, for example, natural or hardened oils, waxes, fats, semisolid or liquid polyols, etc.

The pharmaceutical preparations may additionally also comprise preservatives, solubilizers, viscosity-increasing substances, stabilizers, wetting agents, emulsifiers, sweeteners, colorants, flavourings, salts for altering the osmotic pressure, buffers, coatings or antioxidants. They may also comprise other therapeutically valuable substances.

Subject of the present invention is also the use of the compounds of formula (I) or preferred of formulae (Ic) and (Id) respectively, and their pharmaceutically useful salts for the prevention, delay of progression or the treatment of Alzheimer Disease, malaria or HIV infection.

Subject of the present invention is also the use of the compounds of formula (I) or preferred of formulae (Ic) and (Id), respectively, and their pharmaceutically useful salts for the manufacture of a medication for the prevention, delay of progression or the treatment of Alzheimer Disease, malaria or HIV infection.

Subject of the present invention is also the method for the prevention, delay of progression or the treatment of Alzheimer Disease, malaria or HIV infection, whereby a therapeutically effective dose of a compound of the general formula (I) or preferred of formulae (Ic) and (Id) is applied.

Subject of the present invention is also a pharmaceutical preparation that contains for the inhibition of beta-secretase, cathepsin D, plasmepsin and/or HIV-protease a compound of the general formula (I), or preferred of formulae (Ic) and (Id) as well as commonly used ingredients.

Subject of the present invention is also a pharmaceutical preparation for the prevention, delay of progression or treatment of Alzheimer Disease, malaria and HIV infection that contains a compound of the general formula (I), or preferred of formulae (Ic) and (Id) as well as commonly used ingredients.

The dose may vary within wide limits and has of course to be adapted to the individual circumstances in each individual case. In general, for oral administration, a daily dose of about 3 mg to about 3 g, preferably about 10 mg to about 1 g, for example about 300 mg, per adult (70 kg), divided into preferably 1-3 individual doses which may, for example, be of equal size, may be appropriate, although the upper limit specified may also be exceeded if this should be found to be appropriate; typically, children receive a lower dose according to their age and body weight.

The examples which follow illustrate the present invention. All temperatures are reported in degrees Celsius, pressures in mbar. Unless stated otherwise, the reactions take place at room temperature. The abbreviation "Rf = xx (A)" means, for example, that the Rf value xx is obtained in the solvent system A. The ratio of the solvents relative to one another is always reported in parts by volume. Chemical names of end products and intermediates were obtained with the aid of the program AutoNom 2000 (Automatic Nomenclature).

The following examples are prepared as described in detail in WO2005/070870 pages 24 to 48 which description is herewith incorporated.

### Examples:

1 [1-(5(S)-Amino-6(S)-hydroxy-3,3-dimethyl-7-piperidin-1-yl-heptanoyl)-1,2,3,4-tetrahydro-quinolin-3(R,S)-yl]-carbamic acid methyl ester dihydrochloride
2 1-(5(S)-Amino-6(S)-hydroxy-3,3-dimethyl-7-morpholin-4-yl-heptanoyl)-1,2,3,4-tetrahydro-quinolin-3(R,S)-yl]-carbamic acid methyl ester dihydrochloride
3 {1-[5(S)-Amino-7-(9-aza-bicyclo[3.3.1]non-9-yl)-6(S)-hydroxy-3,3-dimethyl-heptanoyl]-1,2,3,4-tetrahydro-quinolin-3(R,S)-yl}-carbamic acid methyl ester dihydrochloride
4 {1-[5(S)-Amino-7-(cis-2,6-dimethyl-piperidin-1-yl)-6(S)-hydroxy-3,3-dimethyl-heptanoyl]-1,2,3,4-tetrahydro-quinolin-3(R,S)-yl}-carbamic acid methyl ester dihydrochloride
5 {1-[5(S)-Amino-6(S)-hydroxy-3,3-dimethyl-7-(3(R,S)-methyl-piperidin-1-yl)-heptanoyl]-1,2,3,4-tetrahydro-quinolin-3(R,S)-yl}-carbamic acid methyl ester dihydrochloride
6 {1-[5(S)-Amino-6(S)-hydroxy-3,3-dimethyl-7-(4-methyl-piperidin-1-yl)-heptanoyl]-1,2,3,4-tetrahydro-quinolin-3(R,S)-yl}-carbamic acid methyl ester dihydrochloride
7 [1-(5(S)-Amino-7-(S)-sec-butylamino-6(S)-hydroxy-3,3-dimethyl-heptanoyl)-1,2,3,4-tetrahydro-quinolin-3(R,S)-yl]-carbamic acid methyl ester dihydrochloride
8 [1-(5(S)-Amino-7-tert-butylamino-6(S)-hydroxy-3,3-dimethyl-heptanoyl)-1,2,3,4-tetrahydro-quinolin-3(R,S)-yl]-carbamic acid methyl ester dihydrochloride
9 [1-(5(S)-Amino-6(S)-hydroxy-7-isopropylamino-3,3-dimethyl-heptanoyl)-1,2,3,4-tetrahydro-quinolin-3(R,S)-yl]-carbamic acid methyl ester dihydrochloride
10 [1-(5(S)-Amino-7-(R)-sec-butylamino-6(S)-hydroxy-3,3-dimethyl-heptanoyl)-1,2,3,4-tetrahydro-quinolin-3(R,S)-yl]-carbamic acid methyl ester dihydrochloride
11 {1-[5(S)-Amino-7-(cyclopropylmethyl-amino)-6(S)-hydroxy-3,3-dimethyl-heptanoyl]-1,2,3,4-tetrahydro-quinolin-3(R,S)-yl}-carbamic acid methyl ester dihydrochloride
12 [1-(5(S)-Amino-7-cyclopentylamino-6(S)-hydroxy-3,3-dimethyl-heptanoyl)-1,2,3,4-tetrahydro-quinolin-3(R,S)-yl]-carbamic acid methyl ester dihydrochloride
13 [1-(5(S)-Amino-7-benzylamino-6(S)-hydroxy-3,3-dimethyl-heptanoyl)-1,2,3,4-tetrahydro-quinolin-3(R,S)-yl]-carbamic acid methyl ester dihydrochloride
14 {1-[5(S)-Amino-6(S)-hydroxy-7-(2(R)-methoxymethyl-pyrrolidin-1-yl)-3,3-dimethyl-heptanoyl]-1,2,3,4-tetrahydro-quinolin-3(R,S)-yl}-carbamic acid methyl ester dihydrochloride
15 {1-[5(S)-Amino-7-(1-carbamoyl-ethylamino)-6(S)-hydroxy-3,3-dimethyl-heptanoyl-1,2,3,4-tetrahydro-quinolin-3(R,S)-yl}-carbamic acid methyl ester dihydrochloride
16 {1-[7-(3-Acetylamino-pyrrolidin-1-yl)-5(S)-Amino-6(S)-hydroxy-3,3-dimethyl-heptanoyl]-1,2,3,4-tetrahydro-quinolin-3(R,S)-yl}-carbamic acid methyl ester dihydrochloride
17 [1-(5(S)-Amino-7-diethylamino-6(S)-hydroxy-3,3-dimethyl-heptanoyl)-1,2,3,4-tetrahydro-quinolin-3(R,S)-yl]-carbamic acid methyl ester dihydrochloride
18 {1-[5(S)-Amino-7-(2,2-dimethyl-propionylamino)-6(S)-hydroxy-3,3-dimethyl-heptanoyl]-1,2,3,4-tetrahydro-quinolin-3(R,S)-yl}-carbamic acid methyl ester hydrochloride
19 (1-{5(S)-Amino-6(S)-hydroxy-3,3-dimethyl-7-[2-methyl-2-(tetrahydro-pyran-4-yl)-propionylamino]-heptanoyl}-1,2,3,4-tetrahydro-quinolin-3(R,S)-yl)-carbamic acid methyl ester hydrochloride
20 {1-[5(S)-Amino-7-(2-cyclohexyl-2-methyl-propionylamino)-6(S)-hydroxy-3,3-dimethyl-heptanoyl]-1,2,3,4-tetrahydro-quinolin-3(R,S)-yl}-carbamic acid methyl ester hydrochloride
21 (1-{5(S)-Amino-6(S)-hydroxy-3,3-dimethyl-7-[(1-phenyl-cyclobutanecarbonyl)-amino]-heptanoyl}-1,2,3,4-tetrahydro-quinolin-3(R,S)-yl)-carbamic acid methyl ester hydrochloride
22 {1-[5(S)-Amino-7-(2,2-dimethyl-hexanoylamino)-6(S)-hydroxy-3,3-dimethyl-heptanoyl]-1,2,3,4-tetrahydro-quinolin-3(R,S)-yl}-carbamic acid methyl ester hydrochloride
23 [1-(5(S)-Amino-7-{[1-(4-chloro-phenyl)-cyclobutanecarbonyl]-amino}-6(S)-hydroxy-3,3-dimethyl-heptanoyl)-1,2,3,4-tetrahydro-quinolin-3(R,S)-yl]-carbamic acid methyl ester hydrochloride
24 {1-[5(S)-Amino-6(S)-hydroxy-3,3-dimethyl-7-(2-methyl-2-morpholin-4-yl-propionylamino)-heptanoyl]-1,2,3,4-tetrahydro-quinolin-3(R,S)-yl}-carbamic acid methyl ester dihydrochloride
25 (1-{5(S)-Amino-7-[2-(3-fluoro-phenyl)-2-methyl-propionylamino]-6(S)-hydroxy-3,3-dimethyl-heptanoyl}-1,2,3,4-tetrahydro-quinolin-3(R,S)-yl)-carbamic acid methyl ester hydrochloride
26 (1-{5(S)-Amino-7-[(1-cyclohexyl-cyclobutanecarbonyl)-amino]-6(S)-hydroxy-3,3-dimethyl-heptanoyl}-1,2,3,4-tetrahydro-quinolin-3(R,S)-yl)-carbamic acid methyl ester hydrochloride
27 {1-[5(S)-Amino-6(S)-hydroxy-3,3-dimethyl-7-(2-methyl-2-pyridin-3-yl-propionylamino)-heptanoyl]-1,2,3,4-tetrahydro-quinolin-3(R,S)-yl}-carbamic acid methyl ester dihydrochloride
28 {1-[5(S)-Amino-7-(3-chloro-2,2-dimethyl-propionylamino)-6(S)-hydroxy-3,3-dimethyl-heptanoyl]-1,2,3,4-tetrahydro-quinolin-3(R,S)-yl}-carbamic acid methyl ester hydrochloride
29 {1-[7-(2-Acetylamino-2-methyl-propionylamino)-5(S)-Amino-6(S)-hydroxy-3,3-dimethyl-heptanoyl]-1,2,3,4-tetrahydro-quinolin-3(R,S)-yl}-carbamic acid methyl ester hydrochloride
30 (1-{5(S)-Amino-6(S)-hydroxy-3,3-dimethyl-7-[(1-trifluoromethyl-cyclobutanecarbonyl)-amino]-heptanoyl}-1,2,3,4-tetrahydro-quinolin-3(R,S)-yl)-carbamic acid methyl ester hydrochloride
31 {1-[5(S)-Amino-7-(2-cyclohexyloxy-2-methyl-propionylamino)-6(S)-hydroxy-3,3-dimethyl-heptanoyl]-1,2,3,4-tetrahydro-quinolin-3(R,S)-yl}-carbamic acid methyl ester hydrochloride
32 {1-[5(S)-Amino-6(S)-hydroxy-7-(2-methoxy-2-methyl-propionylamino)-3,3-dimethyl-heptanoyl]-1,2,3,4-tetrahydro-quinolin-3(R,S)-yl}-carbamic acid methyl ester hydrochloride
33 {1-[5(S)-Amino-6(S)-hydroxy-3,3-dimethyl-7-(2-methyl-2-piperidin-1-yl-propionylamino)-heptanoyl]-1,2,3,4-tetrahydro-quinolin-3(R,S)-yl}-carbamic acid methyl ester dihydrochloride
34 (1-{5(S)-Amino-6(S)-hydroxy-3,3-dimethyl-7-[(1-methyl-cyclohexanecarbonyl)-amino]-heptanoyl}-1,2,3,4-tetrahydro-quinolin-3(R,S)-yl)-carbamic acid methyl ester hydrochloride
35 (1-{5(S)-Amino-6(S)-hydroxy-7-[2-(1H-indol-3-yl)-2-methyl-propionylamino]-3,3-dimethyl-heptanoyl}-1,2,3,4-tetrahydro-quinolin-3(R,S)-yl)-carbamic acid methyl ester hydrochloride
36 {1-[5(S)-Amino-6(S)-hydroxy-7-(2(R)-methoxy-propionylamino)-3,3-dimethyl-heptanoyl]-1,2,3,4-tetrahydro-quinolin-3(R,S)-yl}-carbamic acid methyl ester hydrochloride
37 (1-{7-[(Adamantane-1-carbonyl)-amino]-5(S)-Amino-6(S)-hydroxy-3,3-dimethyl-heptanoyl}-1,2,3,4-tetrahydro-quinolin-3(R,S)-yl)-carbamic acid methyl ester hydrochloride
38 (1-{5(S)-Amino-7-[(2,2-dimethyl-propionyl)-hydroxy-amino]-6(S)-hydroxy-3,3-dimethyl-heptanoyl}-1,2,3,4-tetrahydro-quinolin-3(R,S)-yl)-carbamic acid methyl ester hydrochloride
39 {1-[5(S)-Amino-7-(3,3-dimethyl-ureido)-6(S)-hydroxy-3,3-dimethyl-heptanoyl]-1,2,3,4-tetrahydro-quinolin-3(R,S)-yl}-carbamic acid methyl ester hydrochloride
40 [1-(5(S)-Amino-7-benzoylamino-6(S)-hydroxy-3,3-dimethyl-heptanoyl)-1,2,3,4-tetrahydro-quinolin-3(R,S)-yl]-carbamic acid methyl ester hydrochloride
41 {1-[7-(Acetyl-methyl-amino)-5(S)-Amino-6(S)-hydroxy-3,3-dimethyl-heptanoyl]-1,2,3,4-tetrahydro-quinolin-3(R,S)-yl}-carbamic acid methyl ester hydrochloride
42 {1-[5(S)-Amino-6(S)-hydroxy-3,3-dimethyl-7-(3,3,3-trifluoro-2(R)-methoxy-2-phenyl-propionylamino)-heptanoyl]-1,2,3,4-tetrahydro-quinolin-3(R,S)-yl}-carbamic acid methyl ester hydrochloride
43 {1-[7-(N-Acetyl-hydrazino)-5(S)-Amino-6(S)-hydroxy-3,3-dimethyl-heptanoyl]-1,2,3,4-tetrahydro-quinolin-3(R,S)-yl}-carbamic acid methyl ester dihydrochloride
44 {1-[5(S)-Amino-6(S)-hydroxy-7-(2-methoxy-3-phenyl-propionylamino)-3,3-dimethyl-heptanoyl]-1,2,3,4-tetrahydro-quinolin-3(R,S)-yl}-carbamic acid methyl ester hydrochloride
45 {1-[5(S)-Amino-7-(3-cyclohexyl-2-methoxy-propionylamino)-6(S)-hydroxy-3,3-dimethyl-heptanoyl]-1,2,3,4-tetrahydro-quinolin-3(R,S)-yl}-carbamic acid methyl ester hydrochloride
46 (1-{5(S)-Amino-6(S)-hydroxy-7-[2-(1H-imidazol-4-yl)-2-methyl-propionylamino]-3,3-dimethyl-heptanoyl}-1,2,3,4-tetrahydro-quinolin-3(R,S)-yl)-carbamic acid methyl ester hydrochloride
47 {1-[5(S)-Amino-7-(2,2-dimethyl-4-methylamino-butyrylamino)-6(S)-hydroxy-3,3-dimethyl-heptanoyl]-1,2,3,4-tetrahydro-quinolin-3(R,S)-yl}-carbamic acid methyl ester dihydrochloride
48 (1-{5(S)-Amino-6(S)-hydroxy-7-[(2(S)-hydroxy-(S)-cyclopentanecarbonyl)-amino]-3,3-dimethyl-heptanoyl}-1,2,3,4-tetrahydro-quinolin-3(R,S)-yl)-carbamic acid methyl ester hydrochloride
49 (1-{5(S)-Amino-6(S)-hydroxy-7-[2-(1,2-dihydro-spiro[3H-indole-3,4'-piperidin]-1'-yl)-2-methyl-propionylamino]-3,3-dimethyl-heptanoyl}-1,2,3,4-tetrahydro-quinolin-3(R,S)-yl)-carbamic acid methyl ester dihydrochloride
50 (1-{5(S)-Amino-6(S)-hydroxy-7-[2-(cis-4-hydroxy-cyclohex-1-yl)-2-methyl-propionylamino]-3,3-dimethyl-heptanoyl}-1,2,3,4-tetrahydro-quinolin-3(R,S)-yl)-carbamic acid methyl ester hydrochloride
51 (1-{5(S)-Amino-6(S)-hydroxy-7-[2-(trans-4-hydroxy-cyclohex-1-yl)-2-methyl-propionylamino]-3,3-dimethyl-heptanoyl}-1,2,3,4-tetrahydro-quinolin-3(R,S)-yl)-carbamic acid methyl ester hydrochloride
52 (1-{5(S)-Amino-6(S)-hydroxy-7-[2-(cis-4-methoxy-cyclohex-1-yl)-2-methyl-propionylamino]-3,3-dimethyl-heptanoyl}-1,2,3,4-tetrahydro-quinolin-3(R,S)-yl)-carbamic acid methyl ester hydrochloride
53 (1-{5(S)-Amino-6(S)-hydroxy-7-[2-(trans-4-methoxy-cyclohex-1-yl)-2-methyl-propionylamino]-3,3-dimethyl-heptanoyl}-1,2,3,4-tetrahydro-quinolin-3(R,S)-yl)-carbamic acid methyl ester hydrochloride
54 {1-[5(S)-Amino-7-(2-cyclohexyl-2(R)-methoxy-acetylamino)-6(S)-hydroxy-3,3-dimethyl-heptanoyl]-1,2,3,4-tetrahydro-quinolin-3(R,S)-yl}-carbamic acid methyl ester hydrochloride
55 {1-[5(S)-Amino-6(S)-hydroxy-7-(2(R)-methoxy-2-phenyl-acetylamino)-3,3-dimethyl-heptanoyl]-1,2,3,4-tetrahydro-quinolin-3(R,S)-yl}-carbamic acid methyl ester hydrochloride
56 {1-[5(S)-Amino-6(S)-hydroxy-7-(2(R)-methoxy-3,3-dimethyl-butyrylamino)-3,3-dimethyl-heptanoyl]-1,2,3,4-tetrahydro-quinolin-3(R,S)-yl}-carbamic acid methyl ester hydrochloride
57 {1-[5(S)-Amino-6(S)-hydroxy-3,3-dimethyl-7-(3,3,3-trifluoro-2-methoxy-2-trifluoromethyl-propionylamino)-heptanoyl]-1,2,3,4-tetrahydro-quinolin-3(R,S)-yl}-carbamic acid methyl ester hydrochloride
58 {1-[5(S)-Amino-6(S)-hydroxy-3,3-dimethyl-7-(3,3,3-trifluoro-2(R)-methoxy-2-methyl-propionylamino)-heptanoyl]-1,2,3,4-tetrahydro-quinolin-3(R,S)-yl}-carbamic acid methyl ester hydrochloride
59 {1-[5(S)-Amino-6(S)-hydroxy-3,3-dimethyl-7-(3,3,3-trifluoro-2(S)-methoxy-2-methyl-propionylamino)-heptanoyl]-1,2,3,4-tetrahydro-quinolin-3(R,S)-yl}-carbamic acid methyl ester hydrochloride
60 {1-[5(S)-Amino-7-(2-cyclohexyl-3,3,3-trifluoro-2(R)-methoxy-propionylamino)-6(S)-hydroxy-3,3-dimethyl-heptanoyl]-1,2,3,4-tetrahydro-quinolin-3(R,S)-yl}-carbamic acid methyl ester hydrochloride
61 {1-[5(S)-Amino-7-(2-phenyl-2(R)-methoxy-propionylamino)-6(S)-hydroxy-3,3-dimethyl-heptanoyl]-1,2,3,4-tetrahydro-quinolin-3(R,S)-yl}-carbamic acid methyl ester hydrochloride
62 {1-[5(S)-Amino-7-(2-cyclohexyl-2(R)-methoxy-propionylamino)-6(S)-hydroxy-3,3-dimethyl-heptanoyl]-1,2,3,4-tetrahydro-quinolin-3(R,S)-yl}-carbamic acid methyl ester hydrochloride
63 (1-{5(S)-Amino-6(S)-hydroxy-7-[(1-methoxy-cyclopentanecarbonyl)-amino]-3,3-dimethyl-heptanoyl}-1,2,3,4-tetrahydro-quinolin-3(R,S)-yl)-carbamic acid methyl ester hydrochloride
64 (1-{5(S)-Amino-6(S)-hydroxy-7-[(1-methoxy-cyclohexanecarbonyl)-amino]-3,3-dimethyl-heptanoyl}-1,2,3,4-tetrahydro-quinolin-3(R,S)-yl)-carbamic acid methyl ester hydrochloride
65 {1-[5(S)-Amino-6(S)-hydroxy-3,3-dimethyl-7-(2-oxo-piperidin-1-yl)-heptanoyl]-1,2,3,4-tetrahydro-quinolin-3(R,S)-yl}-carbamic acid methyl ester hydrochloride
66 {1-[5(S)-Amino-7-(3,3-dimethyl-2-oxo-pyrrolidin-1-yl)-6(S)-hydroxy-3,3-dimethyl-heptanoyl]-1,2,3,4-tetrahydro-quinolin-3(R,S)-yl}-carbamic acid methyl ester hydrochloride
67 {1-[5(S)-Amino-6(S)-hydroxy-7-(4(R)-hydroxy-2-oxo-pyrrolidin-1-yl)-3,3-dimethyl-heptanoyl]-1,2,3,4-tetrahydro-quinolin-3(R,S)-yl}-carbamic acid methyl ester hydrochloride
68 {1-[5(S)-Amino-6(S)-hydroxy-3,3-dimethyl-7-(2-oxo-tetrahydro-pyrimidin-1-yl)-heptanoyl]-1,2,3,4-tetrahydro-quinolin-3(R,S)-yl}-carbamic acid methyl ester hydrochloride
69 {1-[5(S)-Amino-6(S)-hydroxy-3,3-dimethyl-7-(propane-2-sulfonylamino)-heptanoyl]-1,2,3,4-tetrahydro-quinolin-3(R,S)-yl}-carbamic acid methyl ester hydrochloride
70 [1-(5(S)-Amino-7-cyclopropanesulfonylamino-6(S)-hydroxy-3,3-dimethyl-heptanoyl)-1,2,3,4-tetrahydro-quinolin-3(R,S)-yl]-carbamic acid methyl ester hydrochloride
71 [1-(5(S)-Amino-6(S)-hydroxy-3,3-dimethyl-7-phenylmethanesulfonylamino-heptanoyl)-1,2,3,4-tetrahydro-quinolin-3(R,S)-yl]-carbamic acid methyl ester hydrochloride
72 {1-[5(S)-Amino-6(S)-hydroxy-3,3-dimethyl-7-(thiophene-2-sulfonylamino)-heptanoyl]-1,2,3,4-tetrahydro-quinolin-3(R,S)-yl}-carbamic acid methyl ester hydrochloride
73 [1-(5(S)-Amino-7-benzenesulfonylamino-6(S)-hydroxy-3,3-dimethyl-heptanoyl)-1,2,3,4-tetrahydro-quinolin-3(R,S)-yl]-carbamic acid methyl ester hydrochloride
74 {1-[5(S)-Amino-6(S)-hydroxy-3,3-dimethyl-7-(2-methyl-2-piperidin-3(R,S)-yl-propionylamino)-heptanoyl]-1,2,3,4-tetrahydro-quinolin-3(R,S)-yl}-carbamic acid methyl ester dihydrochloride
75 (1-{5(S)-Amino-6(S)-hydroxy-3,3-dimethyl-7-[2-methyl-2-(1-methyl-piperidin-3(R,S)-yl)-propionylamino]-heptanoyl}-1,2,3,4-tetrahydro-auinolin-3(R,S)-yl)-carbamic acid methyl ester dihydrochloride
76 {1-[5(S)-Amino-6(S)-hydroxy-3,3-dimethyl-7-(2-methyl-2-piperidin-2(R,S)-yl-propionylamino)-heptanoyl]-1,2,3,4-tetrahydro-quinolin-3(R,S)-yl}-carbamic acid methyl ester dihydrochloride
77 (1-{5(S)-Amino-6(S)-hydroxy-3,3-dimethyl-7-[2-methyl-2-(1-methyl-piperidin-2(R,S)-yl)-propionylamino]-heptanoyl}-1,2,3,4-tetrahydro-quinolin-3(R,S)-yl)-carbamic acid methyl ester dihydrochloride
78 (1-{5(S)-Amino-6(S)-hydroxy-7-[2(R,S)-(trans-2-hydroxy-cyclohexyl)-2-methyl-propionylamino]-3,3-dimethyl-heptanoyl}-1,2,3,4-tetrahydro-quinolin-3(R,S)-yl)-carbamic acid methyl ester hydrochloride
79 (1-{5(S)-Amino-6(S)-hydroxy-7-[2(R,S)-(3(S)-hydroxy-cyclohex-1(R)-yl)-2-methyl-propionylamino]-3,3-dimethyl-heptanoyl}-1,2,3,4-tetrahydro-quinolin-3(R,S)-yl)-carbamic acid methyl ester hydrochloride
80 {1-[5(S)-Amino-6(S)-hydroxy-7-(2-imidazol-1-yl-2-methyl-propionylamino)-3,3-dimethyl-heptanoyl]-1,2,3,4-tetrahydro-quinolin-3(R,S)-yl}-carbamic acid methyl ester dihydrochloride
81 {1-[5(S)-Amino-7-(2-cyano-2,2-dimethyl-acetylamino)-6(S)-hydroxy-3,3-dimethyl-heptanoyl]-1,2,3,4-tetrahydro-quinolin-3(R,S)-yl}-carbamic acid methyl ester hydrochloride
82 (1-{7-[2-(trans-4-Acetylamino-cyclohexyl)-2-methyl-propionylaminol-5(S)-amino-6(S)-hydroxy-3,3-dimethyl-heptanoyl}-1,2,3,4-tetrahydro-quinolin-3(R,S)-yl)-carbamic acid methyl ester hydrochloride
83 (1-{7-[2-(cis-3-Acetylamino-cyclohexyl)-2-methyl-propionylamino]-5(S)-amino-6(S)-hydroxy-3,3-dimethyl-heptanoyl}-1,2,3,4-tetrahydro-quinolin-3(R,S)-yl)-carbamic acid methyl ester hydrochloride
84 {1-[5(S)-Amino-7-(2,2-difluoro-2-phenyl-acetylamino)-6(S)-hydroxy-3,3-dimethyl-heptanoyl]-1,2,3,4-tetrahydro-quinolin-3(R,S)-yl}-carbamic acid methyl ester hydrochloride
85 {1-[5(S)-Amino-7-(2-cyclohexyl-2,2-difluoro-acetylamino)-6(S)-hydroxy-3,3-dimethyl-heptanoyl]-1,2,3,4-tetrahydro-quinolin-3(R,S)-yl}-carbamic acid methyl ester hydrochloride
86 (1-{5(S)-Amino-7-[2,2-difluoro-2-(tetrahydro-pyran-4-yl)-acetylamino]-6(S)-hydroxy-3,3-dimethyl-heptanoyl}-1,2,3,4-tetrahydro-quinolin-3(R,S)-yl)-carbamic acid methyl ester hydrochloride
87 {1-[5(S)-Amino-6(S)-hydroxy-3,3-dimethyl-7-(2-methyl-propane-2-sulfonylamino)-heptanoyl]-1,2,3,4-tetrahydro-quinolin-3(R,S)-yl}-carbamic acid methyl ester hydrochloride
88 {1-[5(S)-Amino-7-(2-cyclohexyl-propane-2-sulfonylamino)-6(S)-hydroxy-3,3-dimethyl-heptanoyl]-1,2,3,4-tetrahydro-quinolin-3(R,S)-yl}-carbamic acid methyl ester hydrochloride

## Claims

1. Use of a compound of formula 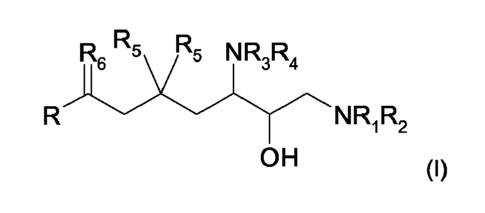 where
R₁ is a) hydrogen, amino or hydroxyl; or
is b) C₁-C₈-alkyl, C₃-C₈-cycloalkyl, C₁-C₈-alkanoyl, C₁-C₈-alkoxycarbonyl, aryl-C₀-C₄-alkyl or heterocyclyl-C₀-C₄-alkyl, which radicals may be substituted by 1-4, C₁-C₈-alkyl, halogen, cyano, oxide, oxo, trifluoromethyl, C₁-C₈-alkoxy, C₁-C₈-alkoxycarbonyl, aryl or heterocyclyl;
R₂ is a) C₁-C₈-alkyl, C₃-C₈-cycloalkyl, C₁-C₈-alkylsulphonyl, C₃-C₈-cycloalkylsulphonyl, aryl-C₀-C₈-alkylsulphonyl, heterocyclylsulphonyl, C₃-C₈-cycloalkyl-C₁-C₈-alkanoyl, aryl-C₁-C₈₋alkanoyl, aryl-C₃-C₈-cycloalkanoyl, C₁-C₈-alkanoyl, C₁-C₈-alkoxycarbonyl, optionally N-mono- or N,N-di-C₁-C₈-alkylated carbamoyl-C₀-C₈-alkyl, aryl-C₀-C₄-alkyl or heterocyclyl-C₀-C₄-alkyl, which radicals may be substituted by 1-4 C₁-C₈-alkyl, C₃-C₁₂-cycloalkyl, C₃-C₈-cycloalkoxy, amino, C₁-C₆-alkylamino, di-C₁-C₆-alkylamino, C₁-C₆-alkanoylamino, C₁-C₆-alkoxycarbonylamino, halogen, oxo, cyano, hydroxyl, oxide, trifluoromethyl, C₁-C₈-alkoxy, optionally N-mono- or N,N-di-C₁-C₈-alkylated carbamoyl-C₀-C₈-alkyl, optionally esterified carboxyl, C₁-C₆-alkylenedioxy, aryl or heterocyclyl; or
is b) together with R₁ and the nitrogen atom to which they are bonded a saturated or partly unsaturated 4-8-membered heterocyclic ring which may contain an additional nitrogen, oxygen or sulphur atom or a -SO- or -SO2- group, in which case the additional nitrogen atom may optionally be substituted by C₁-C₈-alkyl, C₁-C₈-alkanoyl, C₁-C₈-alkoxycarbonyl, aryl or heterocyclyl radicals, and this heterocyclic ring may be part of a bicyclic or tricyclic ring system having a total of up to 16 members and the second ring may also contain a nitrogen, oxygen or sulphur atom or a -SO- or -SO2- group, and the nitrogen atom in the second ring may optionally be substituted by C₁-C₈-alkyl, C₁-C₈-alkanoyl, C₁-C₈-alkoxycarbonyl, aryl or heterocyclyl radicals and all ring systems mentioned may be substituted by 1-4 C₁-C₈-alkyl, C₃-C₈-cycloalkyl, C₁-C₈-alkylsulphonyl, C₃-C₈-cycloalkylsulphonyl, aryl-C₀-C₈-alkylsulphonyl, heterocyclylsulphonyl, C₃-C₈-cycloalkyl-C₁-C₈-alkanoyl, aryl-C₁-C₈-alkanoyl, C₁-C₈-alkanoyl, C₁₋C₈-alkoxycarbonyl, optionally N-mono- or N,N-di-C₁-C₈-alkylated carbamoyl-C₀-C₈-alkyl, halogen, hydroxyl, oxide, oxo, trifluoromethyl, C₁-C₈-alkoxy, C₁-C₈-alkoxy-C₁-C₈-alkoxy, C₁-C₈-alkoxy-C₁-C₈-alkyl, C₁-C₈-alkoxycarbonylamino, C₁-C₈-alkanoylamino, C₁-C₈-alkylamino, N,N-di-C₁-C₈-alkylamino, aryl-C₀-C₄-alkyl, aryloxy-C₀-C₄-alkyl, aryl-C₀-C₄-alkyl-C₁-C₈₋alkoxy, aryloxy-C₀-C₄-alkyl-C₁-C₈-alkoxy, heterocyclyl-C₀-C₄-alkyl, heterocyclyloxy-C₀-C₄₋alkyl, heterocyclyl-C₀-C₄-alkyl-C₁-C₈-alkoxy or heterocyclyloxy-C₀-C₄-alkyl-C₁-C₈-alkoxy;
R₃ is hydrogen, C₁-C₈-alkyl, C₁-C₈-alkoxycarbonyl or C₁-C₈-alkanoyl;
R₄ is hydrogen, C₁-C₈-alkyl, C₁-C₈-alkoxycarbonyl or C₁-C₈-alkanoyl;
R₅ are each independently hydrogen or C₁-C₈-alkyl or, together with the carbon atom to which they are bonded, are a C₃-C₈-cycloalkylidene radical;
R₆ is one oxygen atom or two hydrogen atoms;
R is optionally substituted arylamino, N-aryl-N-((lower alkoxy)(lower alkyl))amino, N-aryl-N-aryl(lower alkyl)amino or heterocyclyl bonded via a ring nitrogen atom;
or pharmaceutically usable salt thereof,
for the preparation of a medication for the inhibition of beta-secretase, cathepsin D, plasmepsin II and/or HIV-protease.

2. Use of a compound of the general formula (I) according to claim 1, where R represents a group of formula 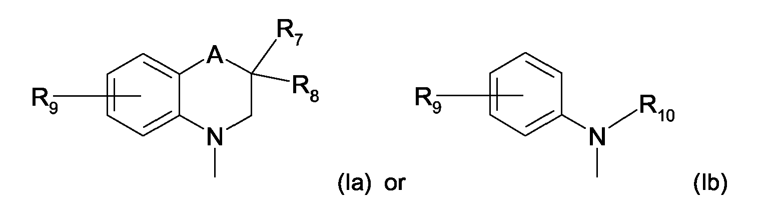 in which
A is a direct bond, methylene, dimethylene, imino, oxy or thio,
R₇ is C₁-C₄-alkoxy-C₁-C₄-alkyl, such as methoxy- or propyloxymethyl, C₃-C₅-alkenyloxy-C₁₋C₄-alkyl, such as allyloxymethyl, C₁-C₄-alkoxy-C₁-C₄-alkoxy-C₁-C₄-alkyl, such as methoxymethoxymethyl or 2-methoxyethoxymethyl, C₁-C₄-alkoxycarbonylamino-C₁-C₄-alkyl, such as methoxy- or ethoxycarbonylaminomethyl, C₁-C₄-alkoxyimino-C₁-C₄-alkyl, such as methoxyiminomethyl, phenyl, C₁-C₄-alkoxycarbonyl, such as methoxycarbonyl, ethoxycarbonyl or isopropyloxycarbonyl, cyano, carbamoyl, N-C₁-C₄-alkylcarbamoyl, such as N-methylcarbamoyl, N-ethylcarbamoyl or N-butylcarbamoyl, C₁-C₄-alkoxy-C₁-C₄₋alkylcarbamoyl, such as N-(2-methoxyethyl)carbamoyl, C₁-C₄-alkoxy such as propyloxy, C₁-C₄-alkoxy-C₁-C₄-alkoxy such as methoxymethoxy or 2-methoxyethoxy, C₁-C₈-alkanoyloxy such as acetoxy, benzoyloxy, N-C₁-C₄-alkylcarbamoylamino, such as N-methylcarbamoylamino, C₁-C₄-alkanoylamino, such as acetylamino, C₁-C₄-alkoxycarbonylamino, such as methoxycarbonylamino, 3- to 6-membered cycloalkylcarbonylamino, such as cyclopropylcarbonylamino, C₁-C₄-alkoxy-C₁-C₄-alkanoylamino, such as methoxyacetylamino, or 5- or 6-membered N,N-(1-oxo(lower alkylene))amino or N,N-(1-oxo-2-oxa(lower alkylene))amino, such as 2-oxopyrrolidin-1-yl or 2-oxooxazolidin-3-yl, N-C₁-C₄-alkylcarbamoylamino, such as methylcarbamoylamino,
R₈ is hydrogen, but may also be C₁-C₄-alkyl such as methyl,
R₉ is hydrogen or halogen and
R₁₀ is C₁-C₄-alkoxy-C₁-C₄-alkyl, such as methoxy-C₁-C₄-alkyl, ethoxy-C₁-C₄-alkyl, propyloxy-C₁-C₄-alkyl, isopropyloxy-C₁-C₄-alkyl, butyloxy-C₁-C₄-alkyl, isobutyloxy-C₁-C₄-alkyl, sec-butyloxy-C₁-C₄-alkyl or tert-butyloxy-C₁-C₄-alkyl, where C₁-C₄-alkyl is, for example, ethyl, propyl or butyl, and is in particular 3-methoxypropyl.

3. Use according to claim 1, of a compound of the general formula 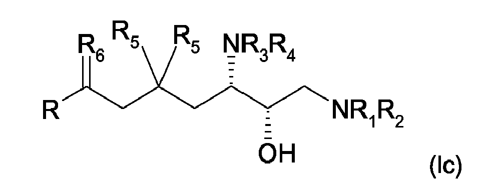 where R, R₁, R₂, R₃, R₄, R₅ and R₆ are each as defined in Claim 1 or pharmaceutically usable salt thereof.

4. Use according to claim 1, of a compound of the general formula 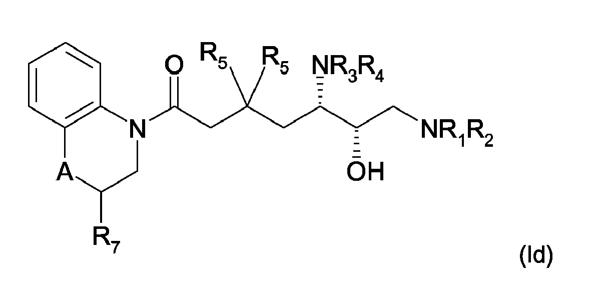 where
A is methylene, oxy or thio,
R₁ is a) hydrogen; or
is b) C₁-C₈-alkyl or C₃-C₈-cycloalkyl;
R₂ is a) C₁-C₈-alkyl, C₃-C₈-cycloalkyl, C₁-C₈-alkanoyl, heterocyclyl-C₁-C₈-alkanoyl, C₃-C₁₂₋cycloalkyl-C₁-C₈-alkanoyl or aryl-C₁-C₈-alkanoyl, which radicals may be substituted by 1 - 4 C₁-C₈-alkyl, C₃-C₈-cycloalkyl C₃-C₈-cycloalkoxy, C₁₋₆-alkylamino, cyano, halogen, hydroxyl, oxide, C₀-C₆-alkylcarbonylamino, C₁-C₈-alkoxy, oxo, trifluoromethyl or aryl; or
b) together with R₁ and the nitrogen atom to which they are bonded, is a saturated or partly unsaturated, 4 - 8-membered heterocyclic ring which may contain an additional nitrogen or oxygen atom, in which case the additional nitrogen atom may optionally be substituted by C₁-C₈-alkyl or C₁-C₈-alkanoyl, and this heterocyclic ring may be part of a bicyclic or tricyclic ring system having a total of up to 16 members, and the second ring may also contain a nitrogen or oxygen atom, in which case the nitrogen atom of the second ring may optionally be substituted by C₁-C₈-alkyl or C₁-C₈-alkanoyl, and all ring systems mentioned may be substituted by 1 - 4 C₁-C₈-alkyl, hydroxyl, cyano, oxide, oxo, C₁-C₈-alkoxy, C₁-C₈-alkoxy-C₁₋C₈-alkoxy, C₀-C₈-alkylcarbonylamino, C₁-C₈-alkoxycarbonylamino or aryloxy-C₀-C₄-alkyl-C₁₋C₈-alkoxy;
R₃ is hydrogen or-(C=O)-C₁-C₄-alkyl;
R₄ is hydrogen;
R₅ are each independently C₁-C₄-alkyl, such as methyl,
R₇ is C₁-C₄-alkoxycarbonylamino such as methoxycarbonylamino, ethoxycarbonylamino, propyloxycarbonylamino, isopropyloxycarbonylamino or butyloxycarbonylamino, C₁-C₄₋alkoxy-C₁-C₄-alkoxy-C₁-C₄-alkyl, such as methoxy-C₁-C₄-alkoxy-C₁-C₄-alkyl, ethoxy-C₁-C₄₋alkoxy-C₁-C₄-alkyl, propyloxy-C₁-C₄-alkoxy-C₁-C₄-alkyl, isopropyloxy-C₁-C₄-alkoxy-C₁-C₄-alkyl or butyloxy-C₁-C₄-alkoxy-C₁-C₄-alkyl, where C₁-C₄-alkoxy is, for example, methoxy, ethoxy, propyloxy or butyloxy, and C₁-C₄-alkyl is, for example, methyl, ethyl, propyl or butyl, in particular methoxymethoxymethyl, 2-methoxyethoxymethyl or 3-methoxypropyloxymethyl, C₁-C₄-alkoxy-C₁-C₄-alkyl, such as methoxy-C₁-C₄-alkyl, ethoxy-C₁-C₄-alkyl, propyloxy-C₁-C₄₋alkyl, isopropyloxy-C₁-C₄-alkyl, butyloxy-C₁-C₄-alkyl, isobutyloxy-C₁-C₄-alkyl, sec-butyloxy-C₁₋C₄-alkyl or tert-butyloxy-C₁-C₄-alkyl, where C₁-C₄-alkyl is, for example, methyl, ethyl, propyl or butyl, in particular ethoxymethyl or 2-methoxyethyl, or N-C₁-C₄-alkylcarbamoyl, such as N-methylcarbamoyl, N-ethylcarbamoyl, N-propylcarbamoyl or N-butylcarbamoyl, or pharmaceutically usable salt thereof.

5. Use of a compound of the general formula (I), (Ic) or (Id), according to the claims 1 to 4 for the preparation of a medication for the prevention, delay of progression or treatment of Alzheimer Disease, malaria or HIV infection.
